# EUROPÄISCHE PATENTSCHRIFT

(11) **EP 0 807 102 B1**
(45) Veröffentlichungstag und Bekanntmachung des Hinweises auf die Patenterteilung: **21.11.2001**
(21) Anmeldenummer: 96901289.7
(22) Anmeldetag: 17.01.1996
(51) Int. Cl.: C07C 251/48, C07D 333/04, C07D 307/02, A01N 37/00

(54) **ALKOXIMINOESSIGSÄUREAMIDE**
ALKOXIMINO ACETIC ACID AMIDES
AMIDES D'ACIDE ALCOXIMINOACETIQUE

(30) Priorität: 30.01.1995 DE 19502813; 01.02.1995 DE 19503162; 24.03.1995 DE 19510770; 25.07.1995 DE 19527099; 20.11.1995 DE 19543199
(43) Veröffentlichungstag der Anmeldung: 19.11.1997
(73) Patentinhaber: BAYER AG, 51368 Leverkusen (DE)
(72) Erfinder: SEITZ, Thomas, D-40764 Langenfeld (DE); HÄNSSLER, Gerd, D-51381 Leverkusen (DE); STENZEL, Klaus, D-40595 Düsseldorf (DE)
(86) Internationale Anmeldenummer: EP9600179
(87) Internationale Veröffentlichungsnummer: WO9623763

(56) Entgegenhaltungen:
- EP-A- 0 528 681
- EP-A- 0 602 514
- WO-A-94/26700
- DATABASE WPI Week 9038 Derwent Publications Ltd., London, GB; AN 286029 XP002002965 & JP,A,02 200 658 ((TSUB) KUMIAI CHEM IND KK) , 8.August 1990

## Beschreibung

Die Erfindung betrifft neue Alkoximinoessigsäureamide, ein Verfahren zu ihrer Herstellung und ihre Verwendung als Schädlingsbekämpfungsmittel.

In WO 94/26700 werden O-Benzyloximetherderivate und deren Verwendung als Schädlingsbekämpfungsmittel beschrieben. Nobuyuki et al. (vgl. JP-A-22 0658) beschreiben N-substituierte Benzylcarboxamid-Derivate und deren Verwendung als Herbizide.

Es wurden neue Verbindungen der allgemeinen Formel (I) gefunden, 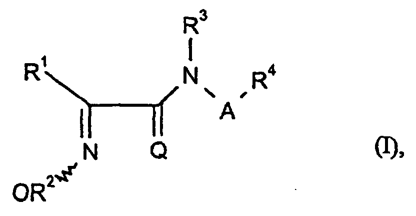 in welcher
- A: für unsubstituiertes geradkettiges Alkylen steht,
- Q: für Sauerstoff oder Schwefel steht,
- R¹: für jeweils gegebenenfalls substituiertes Cycloalkyl, Cycloalkenyl, Aryl oder Heterocyclyl steht,
- R², R³: gleich oder verschieden sind und jeweils fiir Wasserstoff oder jeweils gegebenenfalls substituiertes Alkyl, Alkenyl, Alkinyl oder Cycloalkyl stehen,
- R⁴: für jeweils gegebenenfalls substituiertes Cycloalkyl, Cycloalkenyl, Aryl oder Heterocyclyl steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel.

Ringförmige Verbindungen bilden gegebenenfalls mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Schließlich wurde gefunden, daß die neuen Alkoximinoessigsäureamide der allgemeinen Formel (I) sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z. B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beschrieben und beansprucht.

Gegenstand der Erfindung sind vorzugsweise Verbindungen der Formel (I), in welcher
- A: für unsubstituiertes geradkettiges Alkylen mit 1 bis 6 Kohlenstoffatomen steht,
- Q: für Sauerstoff oder Schwefel steht,
- R¹: für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamio, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
- R²: für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) oder gegebenenfalls substituiertes Phenyl, substituiertes Alkyl mit 1 bis 4 Kohlenstoffatomen, Alkenyl oder Alkinyl mit jeweils 2 bis 4 Kohlenstoffatomen oder Cycloalkyl mit 3 bis 6 Kohlenstoffatomen steht,
- R³: für Wasserstoff oder Alkyl mit 1 bis 4 Kohlenstoffatomen steht,
- R⁴: fiir jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedem steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I), in welcher
- A: für Methylen, 1,2-Ethylen, 1,3-Propylen oder 1,4-Butylen steht,
- Q: für Sauerstoff oder Schwefel steht,
- R¹: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
- R²: für Wasserstoff oder fiir Methyl, Ethyl, n- oder i-Propyl, n-, i-, s- oder t-Butyl oder fiir Benzyl oder Propargyl oder Allyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Besonders bevorzugt sind Verbindungen der Formel (I), in welcher
- A: für Methylen, 1,2-Ethylen, 1,3-Propylen oder 1,4-Butylen steht,
- Q: für Sauerstoff oder Schwefel steht,
- R¹: für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl,
Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
- R²: für Wasserstoff, Methyl, Ethyl, n-, i-Propyl, n-, i- oder t-Butyl oder fiir Benzyl oder Allyl steht,
- R³: für Wasserstoff oder für Methyl steht,
- R⁴: für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl,
Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für Methylen, 1,2-Ethylen oder 1,3-Propylen steht,
- Q: für Sauerstoff steht,
- R¹: für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder fiir Naphthyl, Benzofuranyl oder Benzothienyl steht,
- R²: für Wasserstoff, Methyl oder Ethyl, fiir n- oder i-Propyl, für n-, i-, s- oder t-Butyl oder für Benzyl oder Allyl steht,
- R³: für Wasserstoff oder Methyl steht,
- R⁴: für Cyclohexyl oder gegebenenfalls einfach bis dreifach substituiertes Phenyl, Thienyl, Furyl, Benaofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluolmethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Von ganz besonderem Interesse sind Verbindungen der Formel (I), in welcher der Rest R¹ für unsubstituiertes oder in 3- und/oder 4-Stellung substituiertes Phenyl, oder für unsubstituiertes oder in 4- und/oder 5-Stellung substituiertes Furanyl oder Thienyl steht, wobei als Substituenten die oben genannten und insbesondere Chlor, Brom, Fluor, Nitro, Methylsulfonyl, Phenyl, Phenoxy, Benzyloxy, Cyclopropyl, Cyclohexyl, Methyl, Ethyl, n-, i-Propyl, n-, i-, s-, t-Butyl, n-Pentyl, n-Hexyl, n-Heptyl, Methoxy, Ethoxy, Methylthio, Trifluormethyl und Trifluormethoxy ausgewählt sind oder jeweils gegebenenfalls durch Fluor substituiertes 3,4-Methylen- und Ethylendioxo, Propan-1,3-diyl und Butan-1,4-diyl substituiertes Phenyl oder in 2-Stellung substituiertes Naphthyl, Benzofuranyl oder Benzothienyl bedeuten.

Insbesondere sind auch Verbindungen der Formel (I) bevorzugt, in denen
- R²: für Methyl oder Ethyl steht.

Insbesondere sind auch Verbindungen der Formel (I) bevorzugt, in denen
- R³: für Wasserstoff steht.

Insbesondere sind auch Verbindungen der Formel (I) bevorzugt, in denen R⁴ fiir in 3- und 4-Stellung durch Methoxy substituiertes Phenyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Bevorzugte Einzelverbindungen können den folgenden Tabellen entnommen werden:

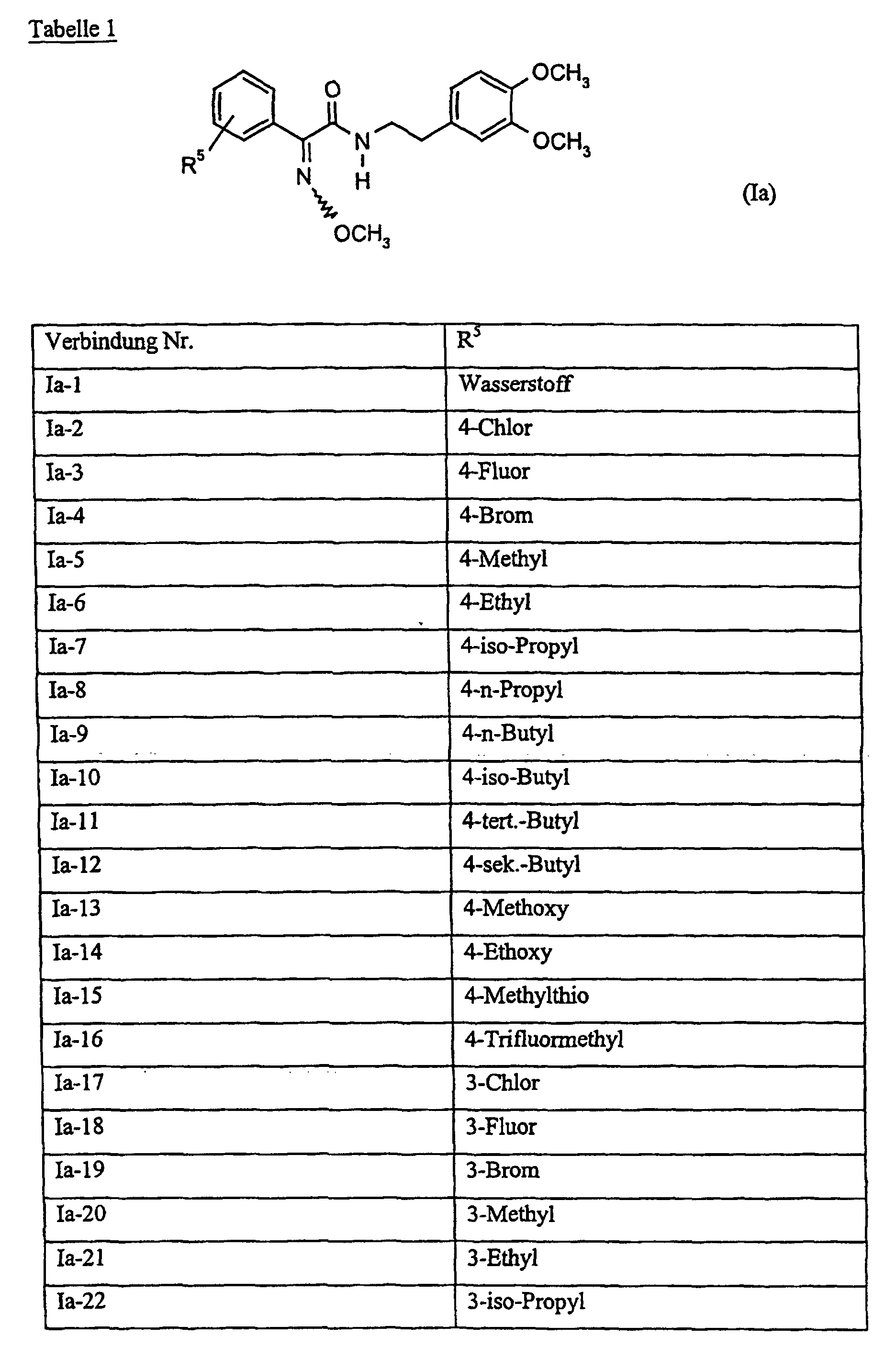

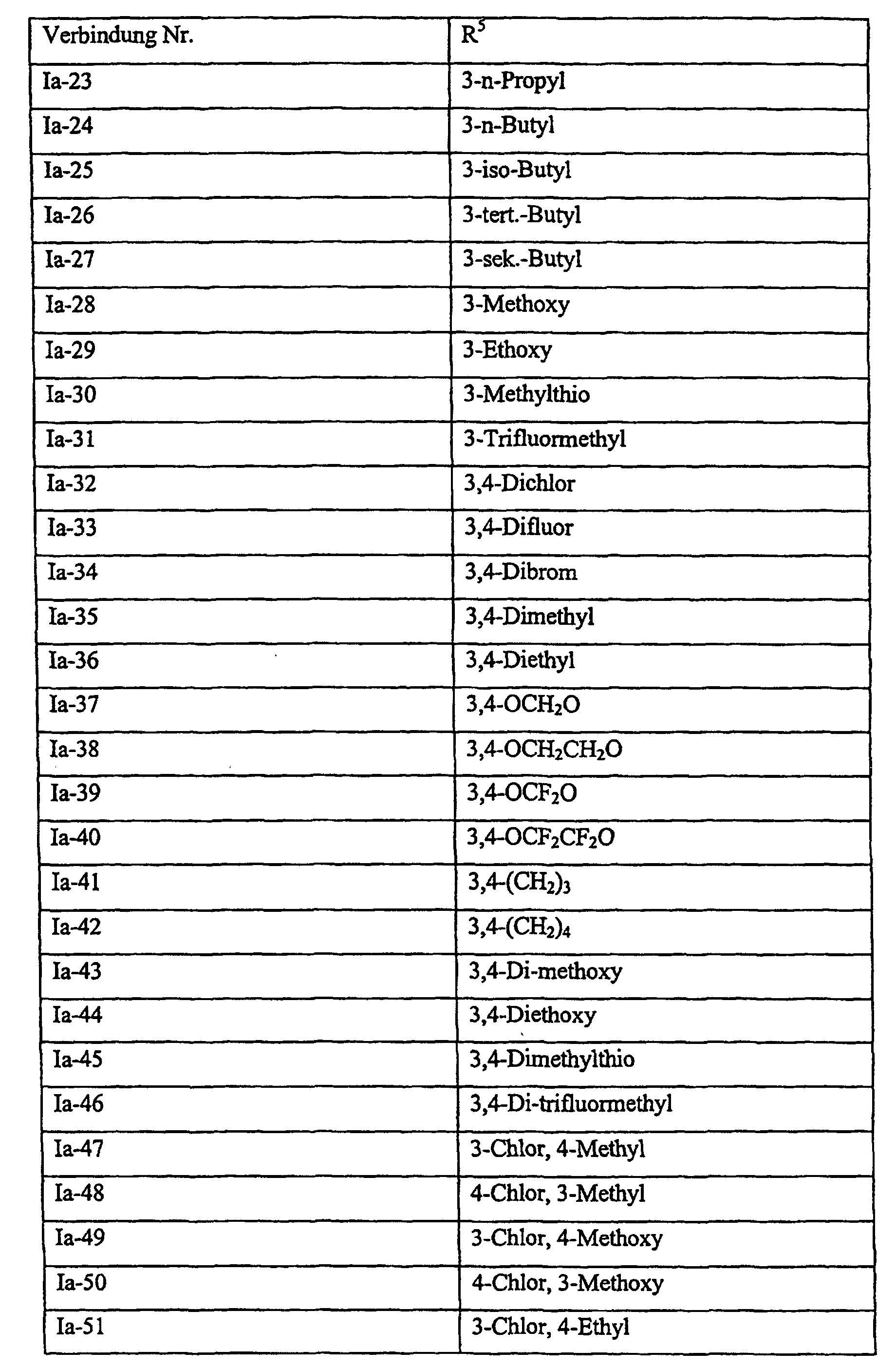

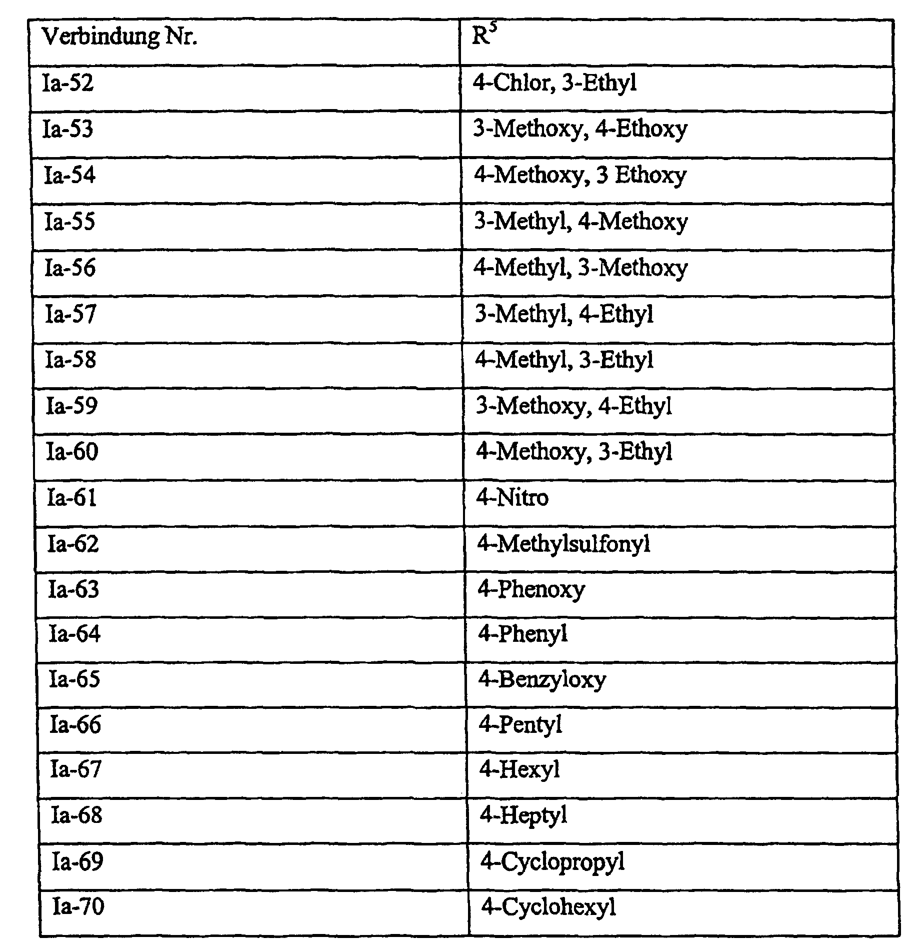

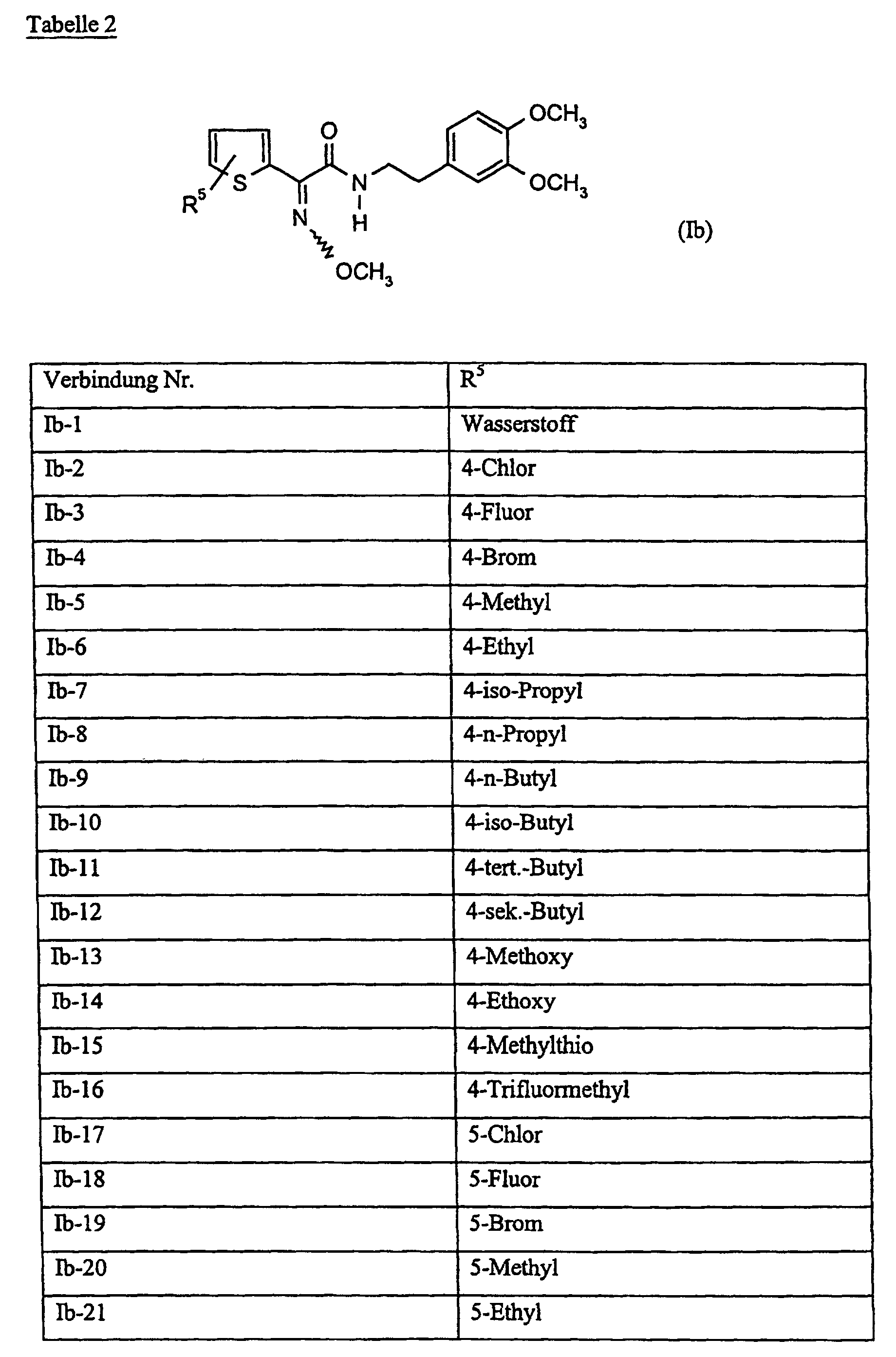

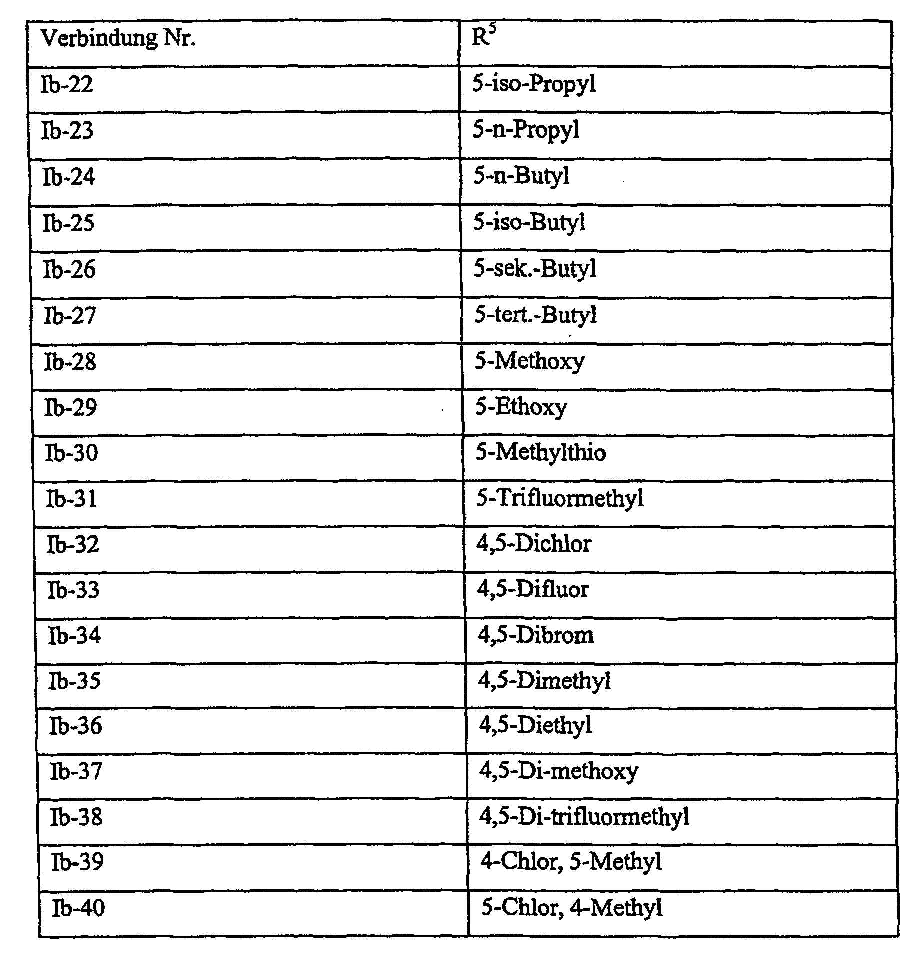

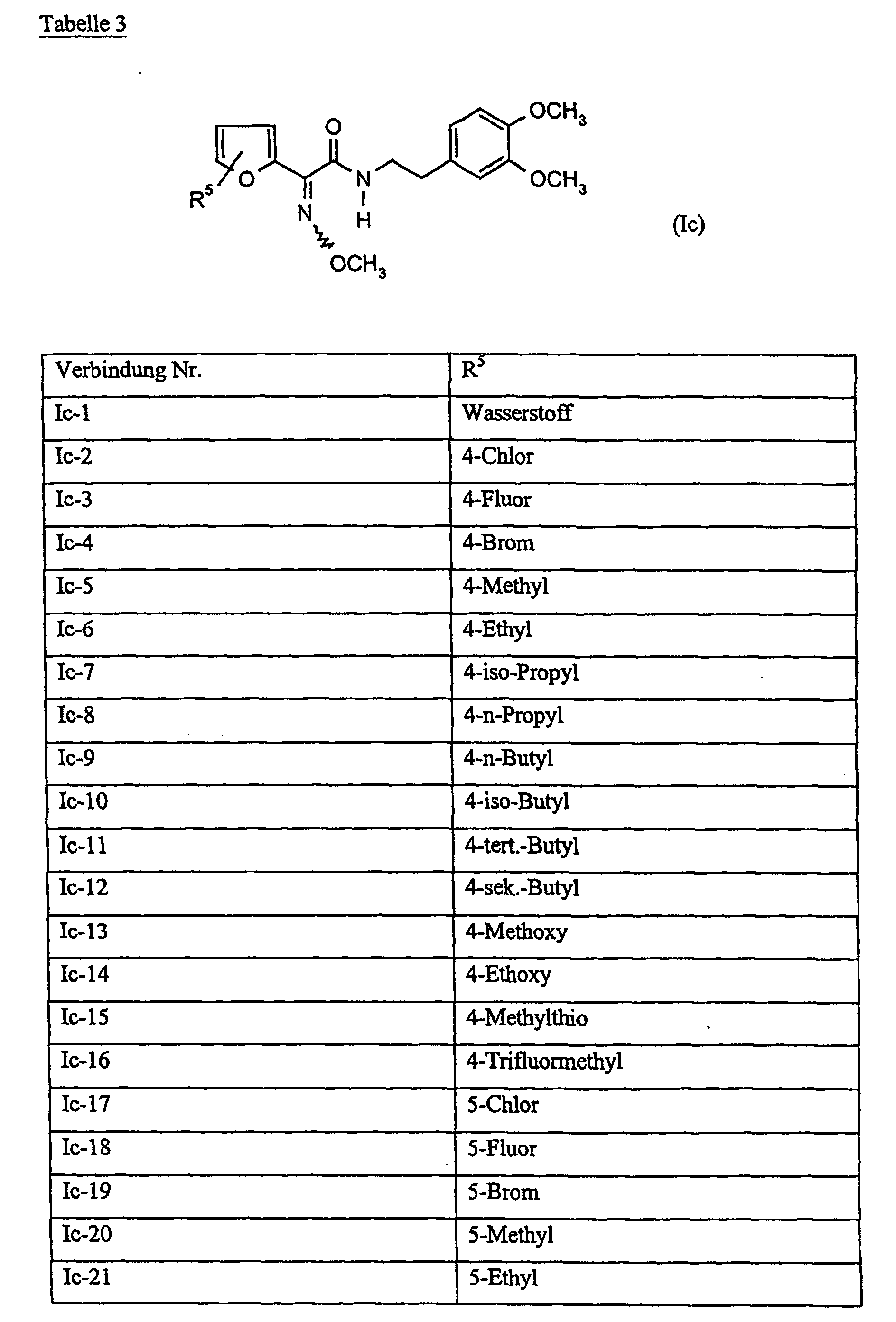

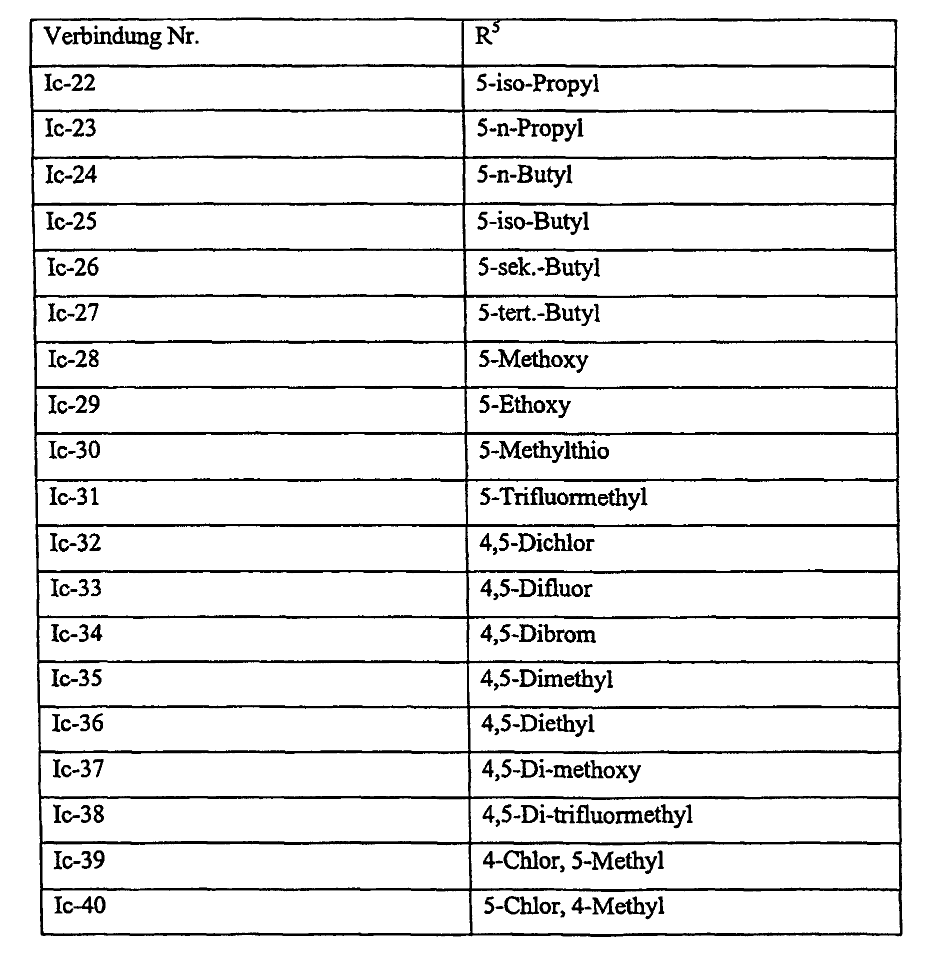

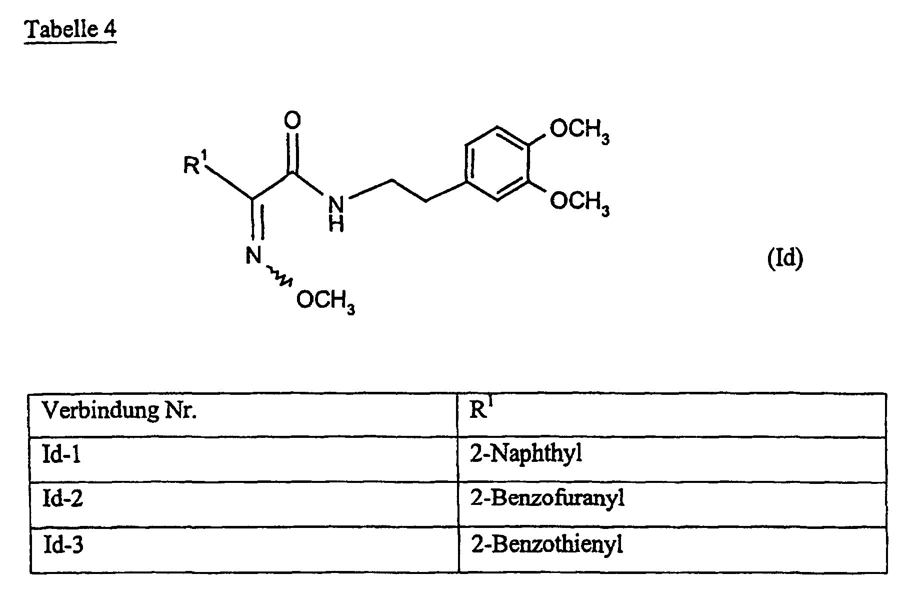

### Tabelle 5

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁴) ein Phenylrest steht, der die in den Verbindungen Ia-1 bis Ia-70 als R⁵ jeweils angegebenen Substituenten trägt.

### Tabelle 6

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁴) einer der folgenden dreifach substituierten Phenylreste steht:
Substituenten: 3,4,5-Trimethoxy; 3,4,5-Trichlor, 3,4,5-Trimethyl.

### Tabelle 7

Verbindungen Ia-1 bis Id-3 entsprechend den Formeln Ia, Ib, Ic und Id, wobei anstelle der Methoximino-Gruppe (im allgemeinen bezeichnet als R²) eine Ethoximino-Gruppe steht, die die in den Verbindungen Ia-1 bis Ia-70 als R⁵ die jeweils angegebenen Substituenten trägt.

Weiter wurde gefunden, daß man die neuen Alkoximinoessigsäureamide der allgemeinen Formel (I) erhält, wenn man Carbonsäurederivate der allgemeinen Formel (II) 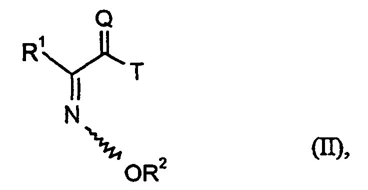 in welcher
- R¹, R² und Q: die oben angegebene Bedeutung haben und
- T: fiir Hydroxy, Halogen oder Alkoxy steht,
mit einem Amin der allgemeinen Formel (III) 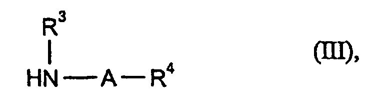 in welcher
- R³, R⁴ and A: die oben angegebene Bedeutung haben,
- oder mit einem Hydrogenhalogenid hiervon - gegebenenfalls in Gegenwart eines Säureakzeptors, gegebenenfalls in Gegenwart eines Kondensationsmittels und gegebenenfalls in Gegenwart eines Verdünnungsmittels umsetzt.

Die zur Durchführung des erfindungsgemäßen Verfahrens als Ausgangsstoffe benötigten Carbonsäurederivate sind durch die Formel (II) allgemein definiert. In dieser Formel (II) haben (Q, R¹ und R²) vorzugsweise insbesondere diejenige Bedeutungen, die bereits im Zusammenhang mit der Beschreibung der erfindungsgemäßen Verbindungen der Formel (I) als bevorzugt bzw. als insbesondere bevorzugt für (Q, R¹ und R²) angegeben wurden; T steht vorzugsweise für Alkoxy mit 1 bis 4 Kohlenstoffatomen, insbesondere für Methoxy oder Ethoxy, fiir Hydroxy oder Chlor.

Die Ausgangsstoffe der Formel (II) sind bekannt und/oder können nach an sich bekannten Verfahren hergestellt (vgl. EP-A 178 826, EP-A 242 081, EP-A 382 375, EP-A 493 711, EP-A 432 503, DE-A 3 938 054).

Die weiter als Ausgangsstoffe zu verwendeten Amine sind durch die Formel (III) allgemein definiert.

Die Ausgangsstoffe der Formel (III) sind bekannte organische Synthesechemikalien und/oder können nach an sich bekannten Verfahren hergestellt werden.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren wird gegebenenfalls in Gegenwart eines Verdünnungsmittels durchgeführt. Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethyl- oder -diethylether, Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens setzt man je Mol an Carbonsäurederivat der Formel (II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens in einem größeren Bereich variiert werden. Im allgemeinen arbeitet man bei Temperaturen zwischen -78°C und +120°C, vorzugsweise bei Temperaturen zwischen -60°C und +25°C.

Zur Durchführung des erfindungsgemäßen Verfahrens zur Herstellung der Verbindungen der Formel (I) setzt man pro Mol des Alkoximinoessigsäurederivates der Formel (II) im allgemeinen 0,5 bis 5 Mol, vorzugsweise äquimolare Mengen an Amin der Formel (III) ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren kann auch als zweistufiger Prozess durchgeführt werden. Dabei werden die Carbonsäurederivate der allgemeinen Formel (II) zunächst in eine aktivierte Form überführt und in einem anschließenden Schritt mit den Aminen der allgemeinen Formel (III) zu den erfindungsgemäßen Alkoximinoessigsäurederivaten der allgemeinen Formel (I) umgesetzt.

Als aktivierte Form der Carbonsäurederivate der Formel (II) kommen alle Carboxyaktivierten Derivate infrage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Carbonsäuren.

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Amen, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Altemaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Phytophthora-Arten eingesetzt.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen fiir Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

### Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb,
Cymoxanil, Cyproconazole, Cyprofuram,
Dichlorophen, Diclobutrazol, Diclofluanid, Diclomezin, Dicloran, Diethofencarb,
Difenoconazol, Dimethirimol, Dimethomorph, Diniconazol, Dinocap, Diphe-nylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Ipiodion, Isoprothiolan,
Kasugamycin, Kupfer-Zubereitungen, wie: Kupferhydroxid, Kupfemaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157
419, CGA 184699, Chloethocarb, Chlorethoxyfos, Chloretoxyfos, Chlorfenvinphos,
Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb,
Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lambda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Pirimiphos A, Profenofos,
Profenophos, Promecarb, Propaphos, Propoxur, Prothiofos, Prothiophos, Prothoat,
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.
Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffinengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1:

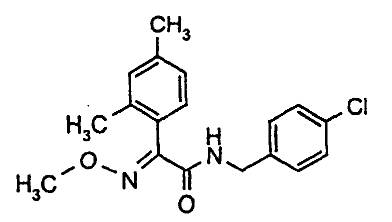

Eine Mischung aus 1,66 g (8 mmol) 2-Methoximino-2-[2-(2,4-dimethyl)-phenyl]-essigsäuremethylester und 1,14 g (8 mmol) 4-Chlorbenzylamin wird in der Schmelze 12 Stunden bei 120°C gerührt. Nach Abkühlen wird in Dichlormethan aufgenommen, einmal mit Wasser, dann mit IN Salzsäure und dann wieder mit Wasser gewaschen, über Natriumsulfat getrocknet und filtriert. Das Filtrat wird im Vakuum eingeengt und der Rückstand mit Petrolether/Essigester (5:1) an Kieselgel chromatografiert.

Man erhält 1,24 g (47 % der Theorie) N-(4-Chlorbenzyl)-2-methoximino-2-[2-(2,4-dimethyl)-phenyl]-essigsäureamid als Öl. ¹H-NMR (CDCl₃, TMS): δ = 3,95 ppm (s,3H)

### Beispiel 2:

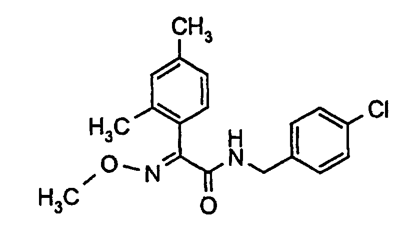

Zu einer Lösung von 4,14 g (20 mol) 2-Methoximino-2-[2-(2,4-dimethyl)-phenyl]-essigsäure in 80 ml Methylenchlorid tropft man nacheinander bei -10°C 2,8 ml (20 mmol) Triethylamin und 2,8 g (20 mmol) Chlorameisensäureisobutylester zu und rührt 2 Stunden bei -10 °C. Dann tropft man eine Lösung von 2,55 g (20 mmol) 4-Chloranilin in 10 ml Methylenchlorid innerhalb von 30 Minuten zu, rührt 2 Stunden bei -10 °C und noch 18 Stunden bei 20 °C. Das Gemisch wird im Vakuum eingeengt, in Dichlormethan aufgenommen, einmal mit Wasser, dann mit IN Salzsäure und dann wieder mit Wasser gewaschen, über Natriumsulfat getrocknet, filtriert und wieder eingeengt. Der Rückstand wird mit Diisopropylether verrührt, die so erhaltenen Kristalle werden abfiltriert und getrocknet.

Man erhält 3,8 g (60 % der Theorie) N-4-Chlorphenyl-2-methoximino-2-[2-(2,4-dimethyl)-phenyl]-essigsäureamid vom Schmelzpunkt 121°C.

### Beispiel 3

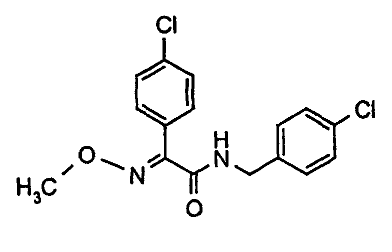

Es werden 4,45 g (20 mmol) 2-Methoximino-2-(4-chlorphenyl)-essigsäuremethylester in 50 ml Methanol vorgelegt und nacheinander bei Raumtemperatur 2,83 g (20 mmol) 4-Chlorbenzylamin und 7,2 g (40 mmol) 30%ige methanolische Natriummethylat-Lösung dazugegeben. Die Mischung wird 20 h bei 65°C gerührt, danach das Lösungsmittel abdestilliert, der Rückstand in Methylenchlorid aufgenommen, nacheinander mit Wasser, verdünnter Salzsäure und Wasser gewaschen und über Natriumsulfat getrocknet. Nach Abdestillieren des Lösungsmittels wird mit Petrolether/Essigester (10:1) an Kieselgel chromatographiert.

Man erhält 3,4 g (51 d. Theorie) N-(4-Chlorbenzyl)-2-methoximino-2-(4-chlorphenyl)essigsäureamid.
¹H-NMR (DCDl₃, TMS): δ = 3,98 ppm (s,3H)

Analog den Beispielen 1 bis 3, sowie entsprechend der allgemeinen Beschreibung des erfindungsgemäßen Herstellungsverfahrens, können beispielsweise auch die in der nachstehenden Tabelle 7 aufgeführten Verbindungen hergestellt werden:

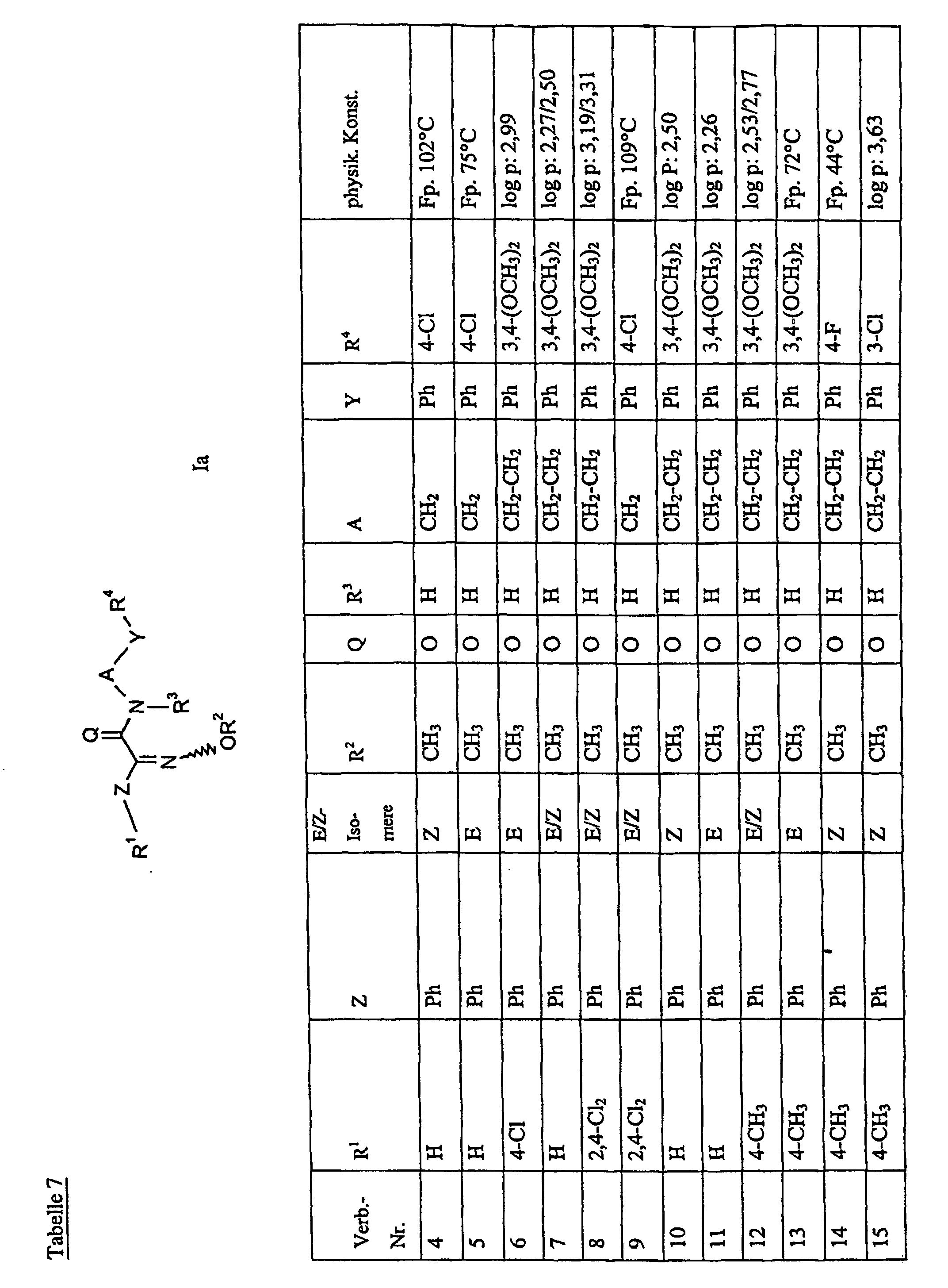

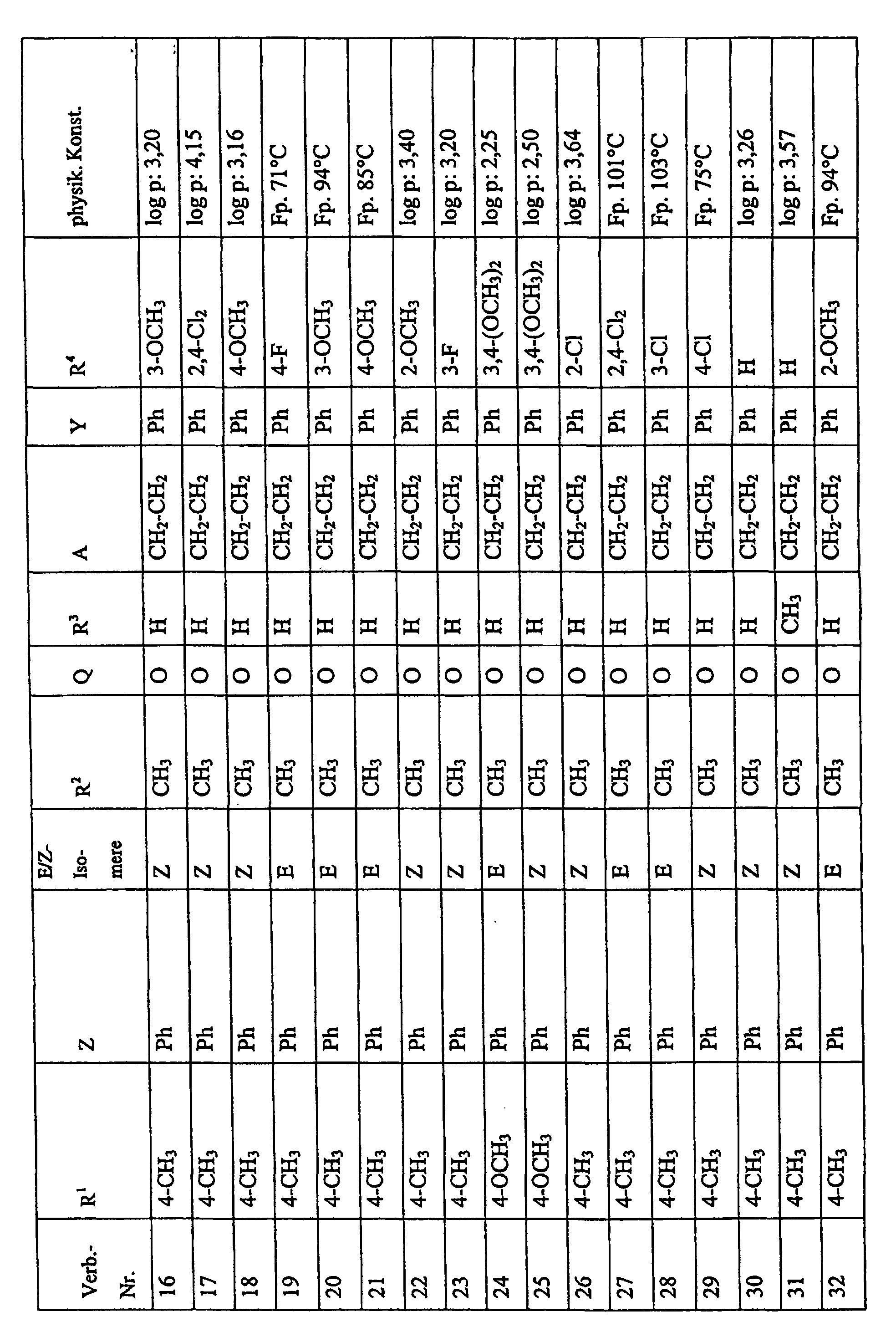

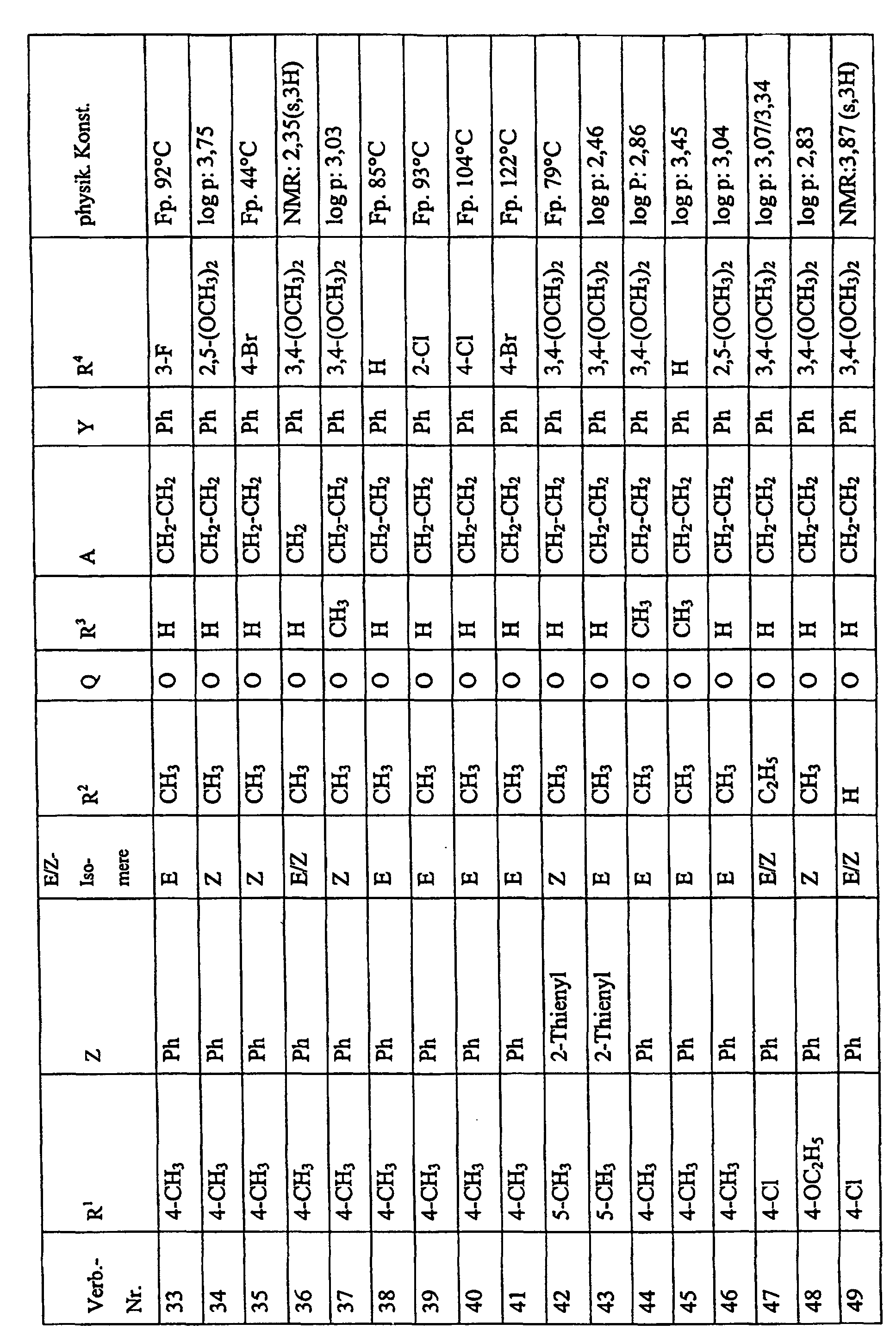

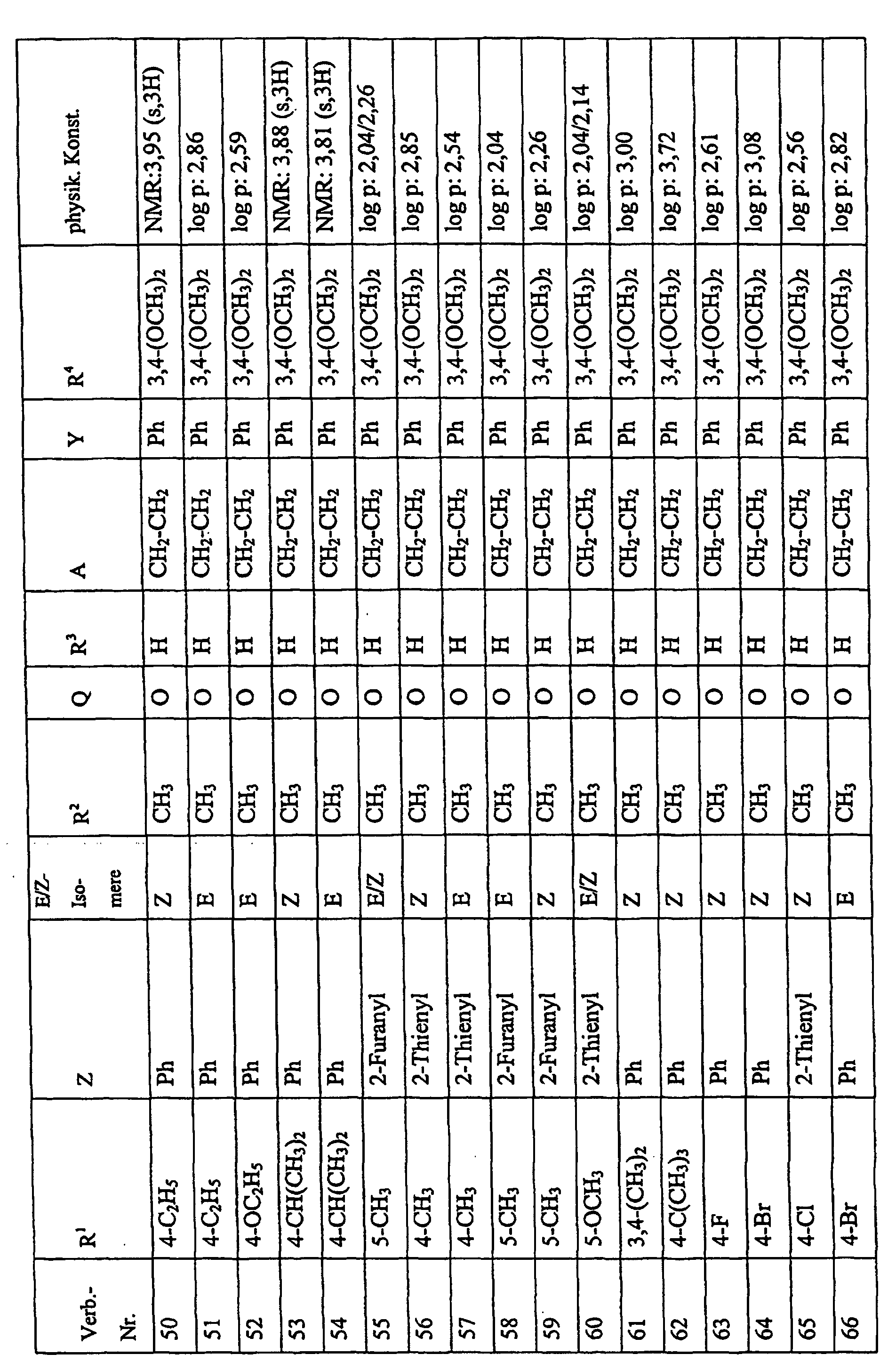

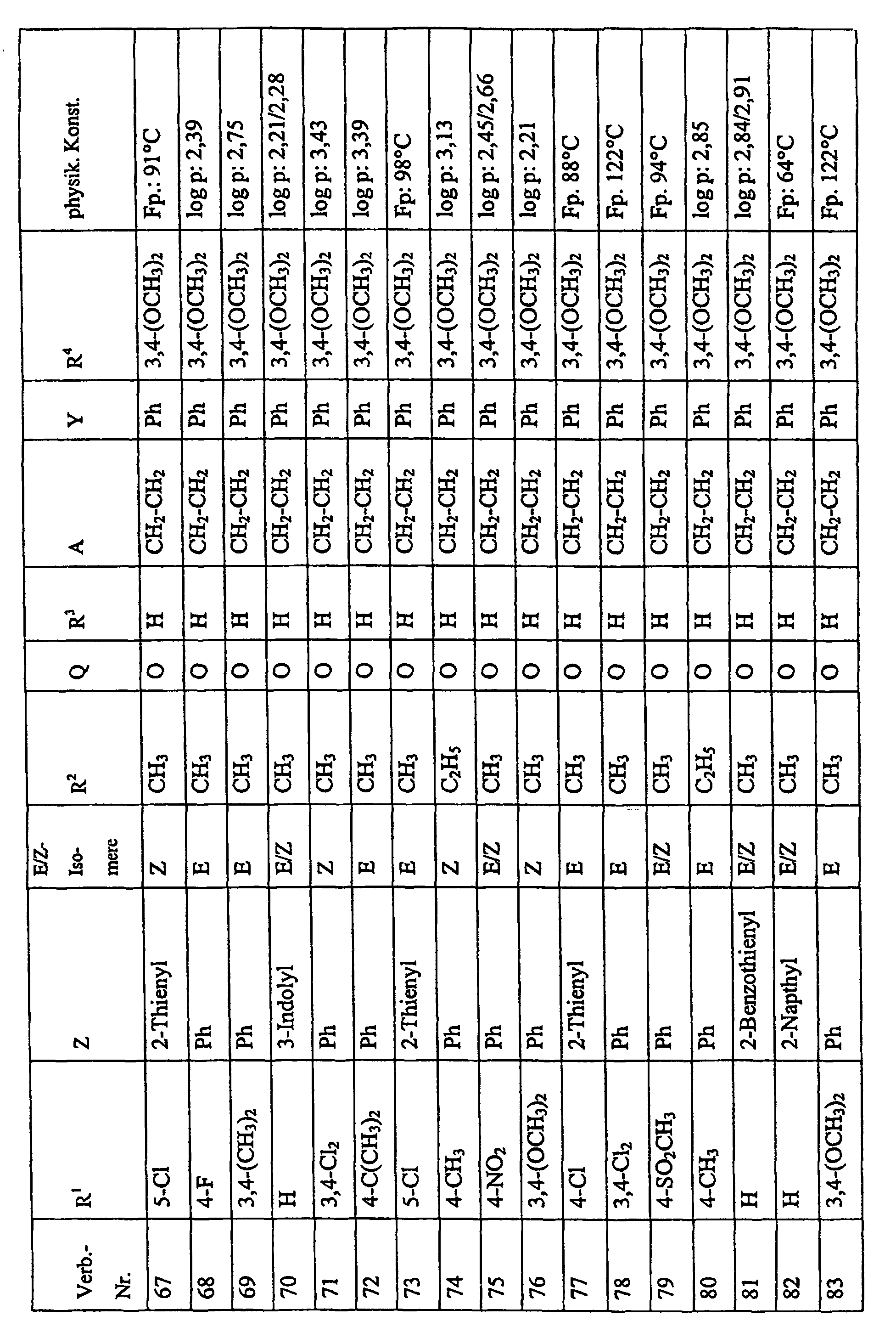

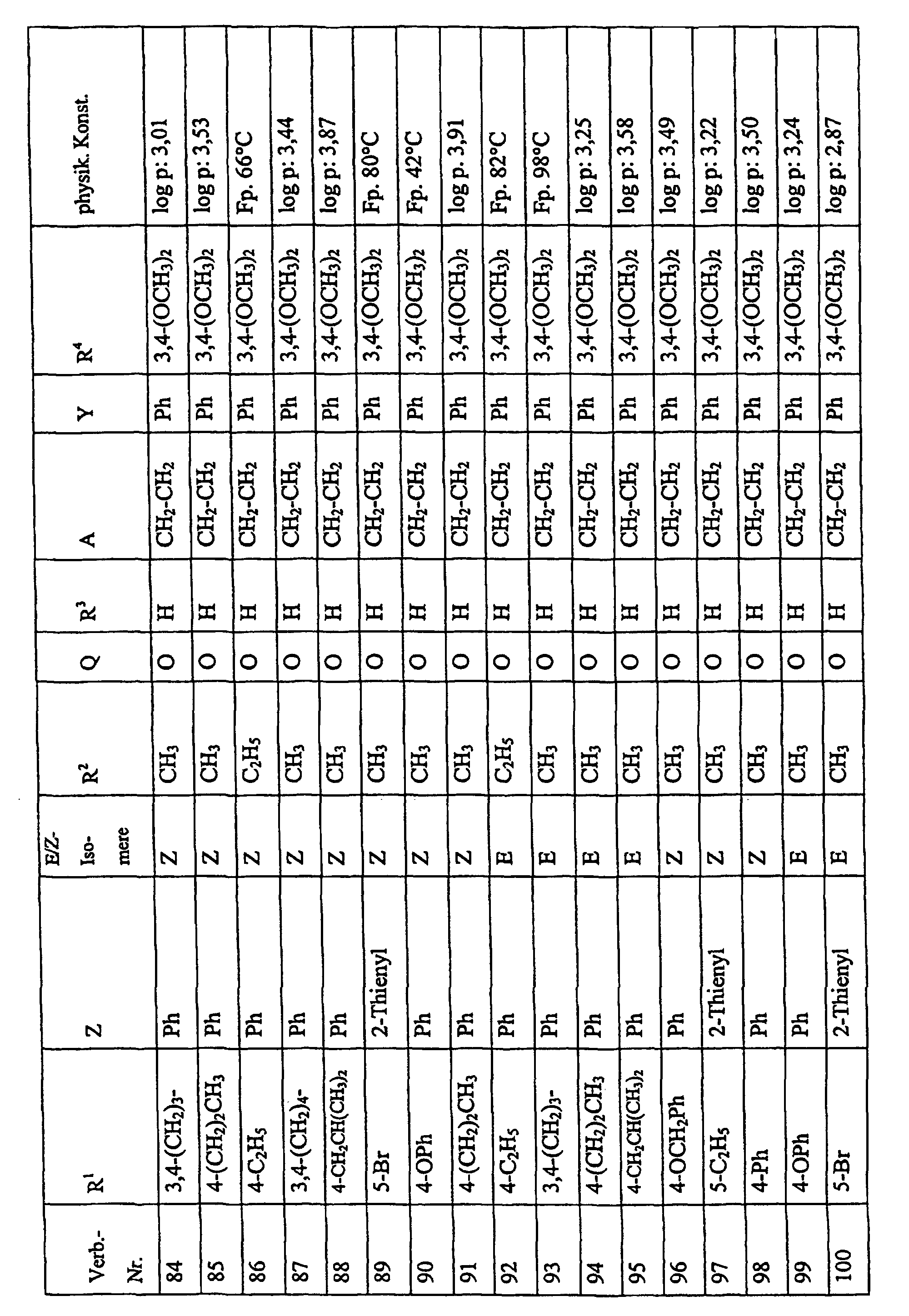

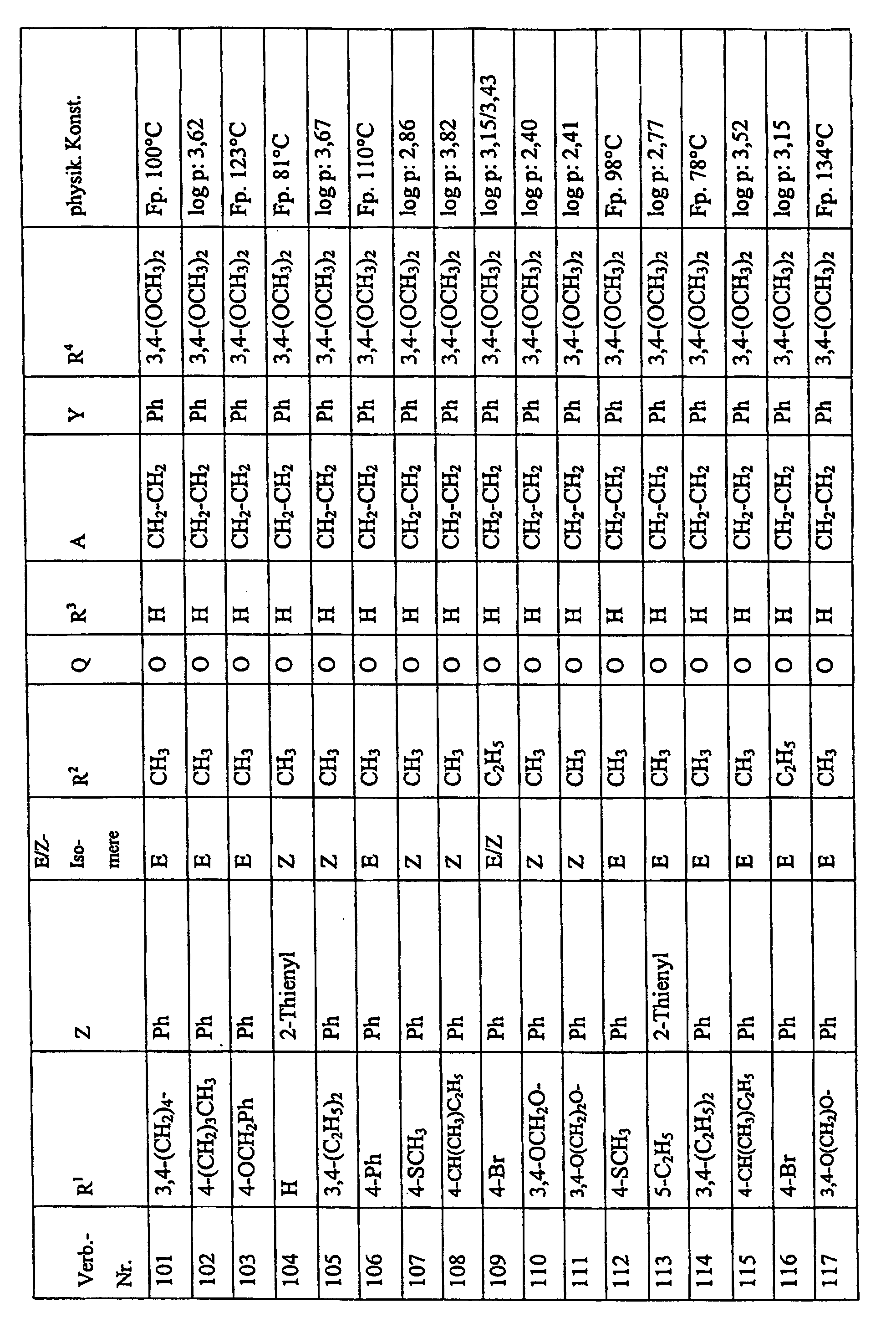

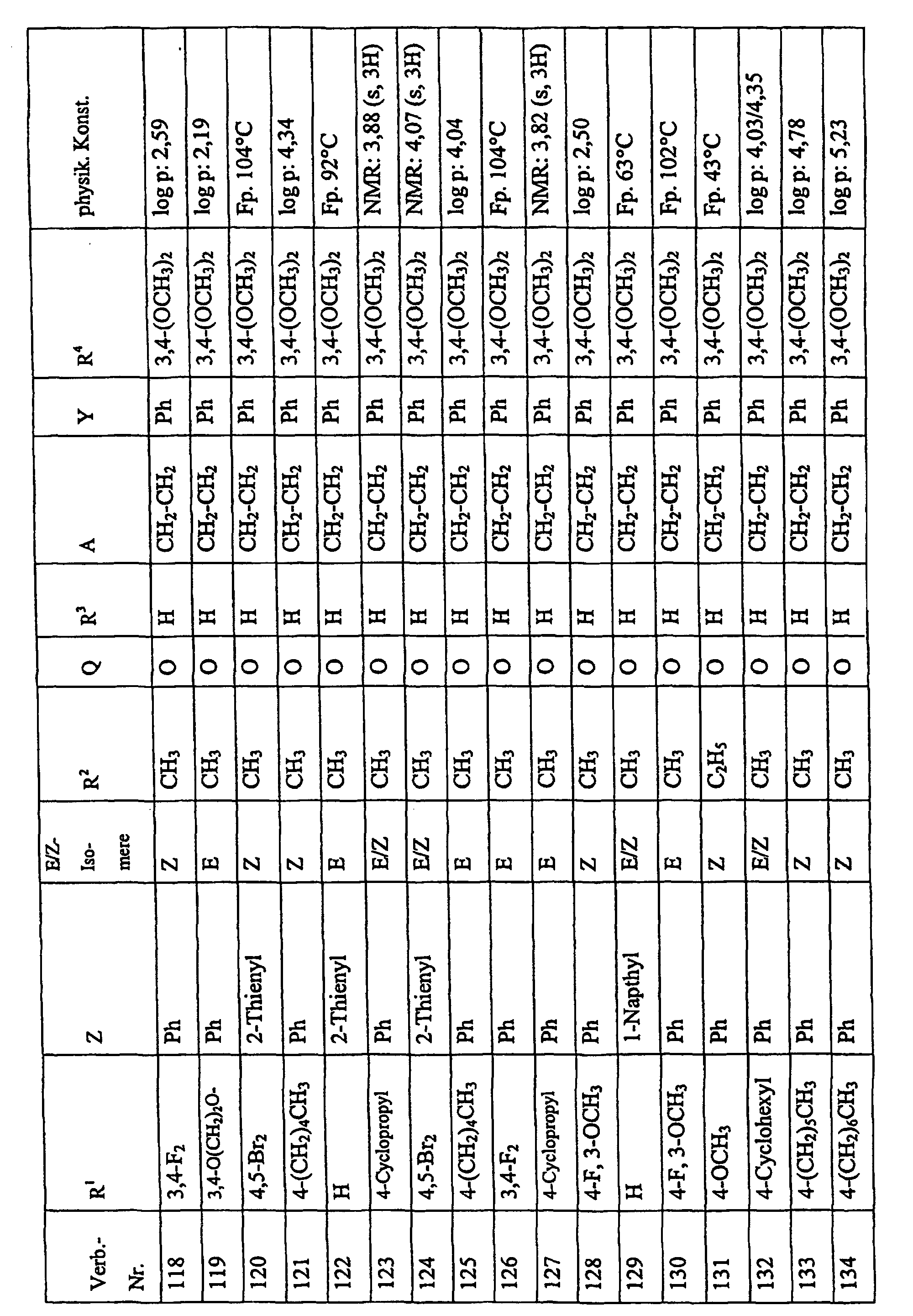

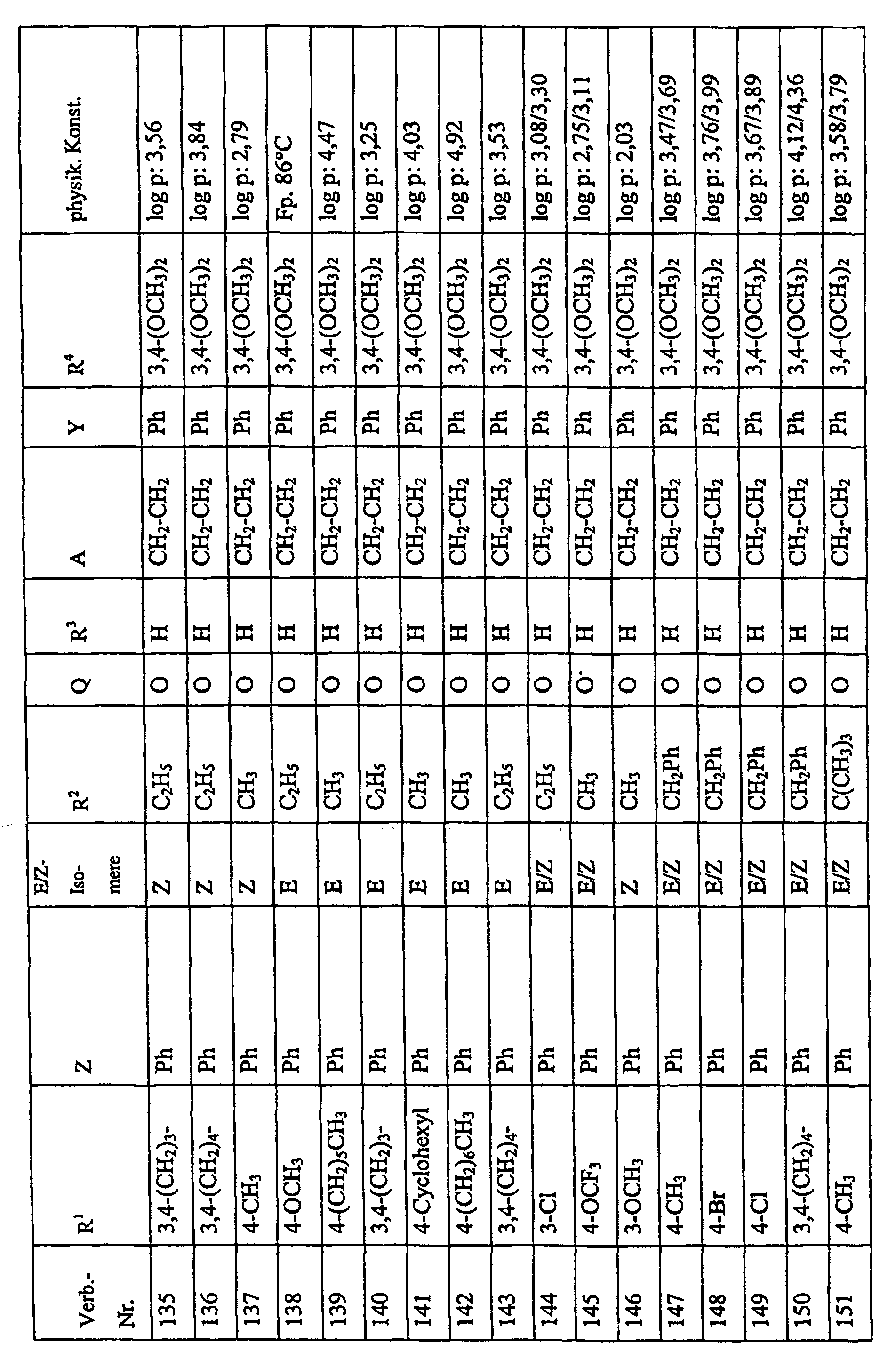

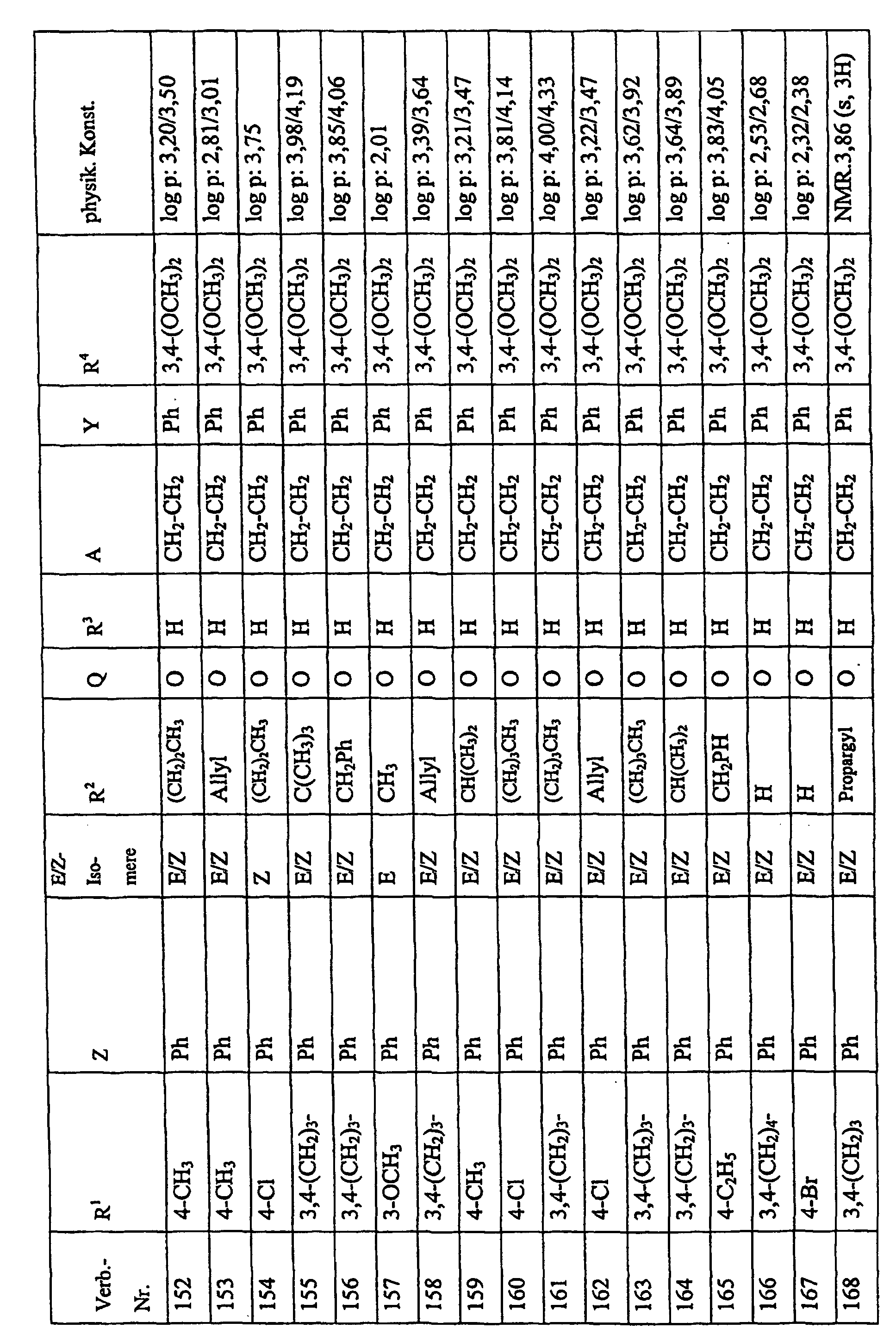

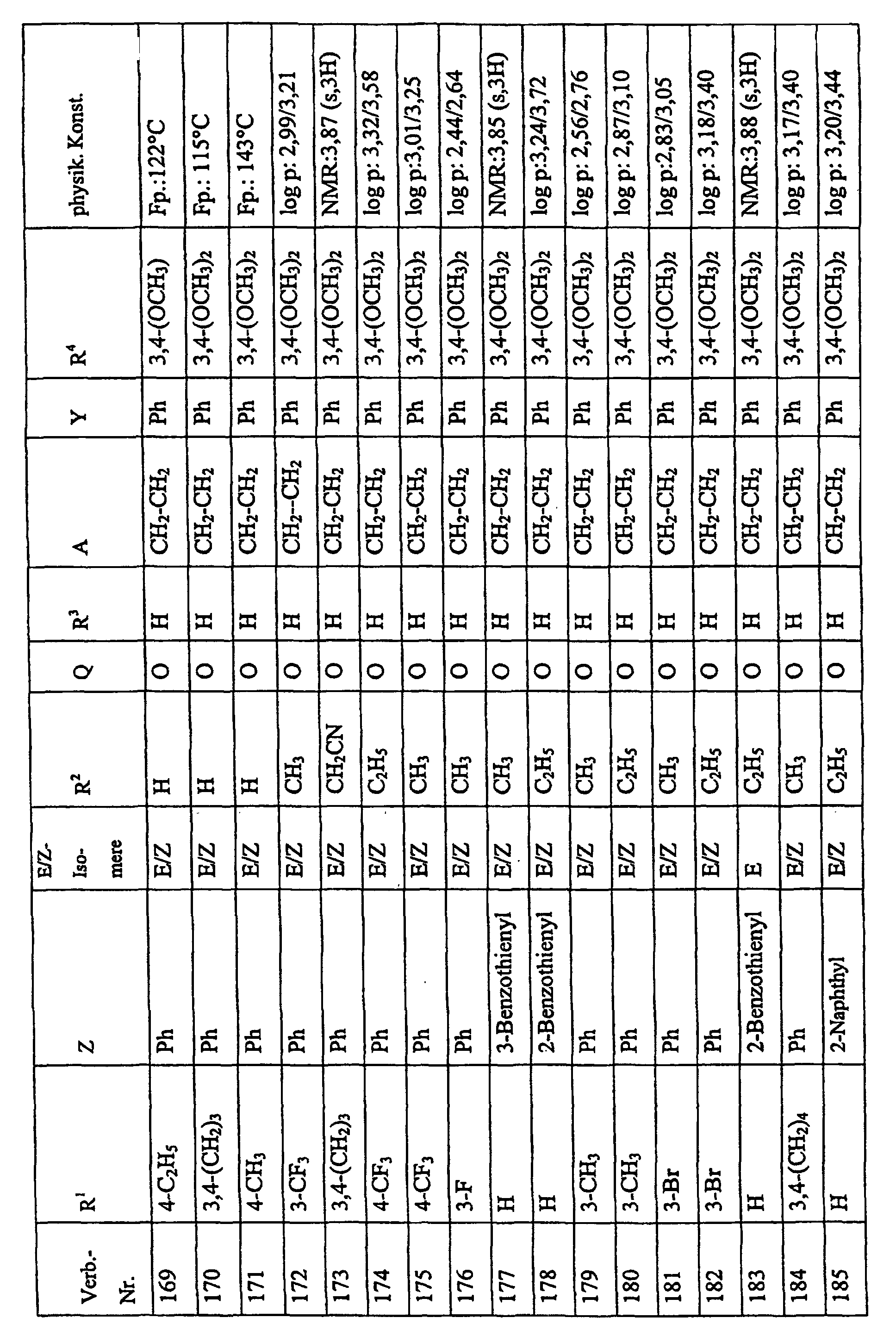

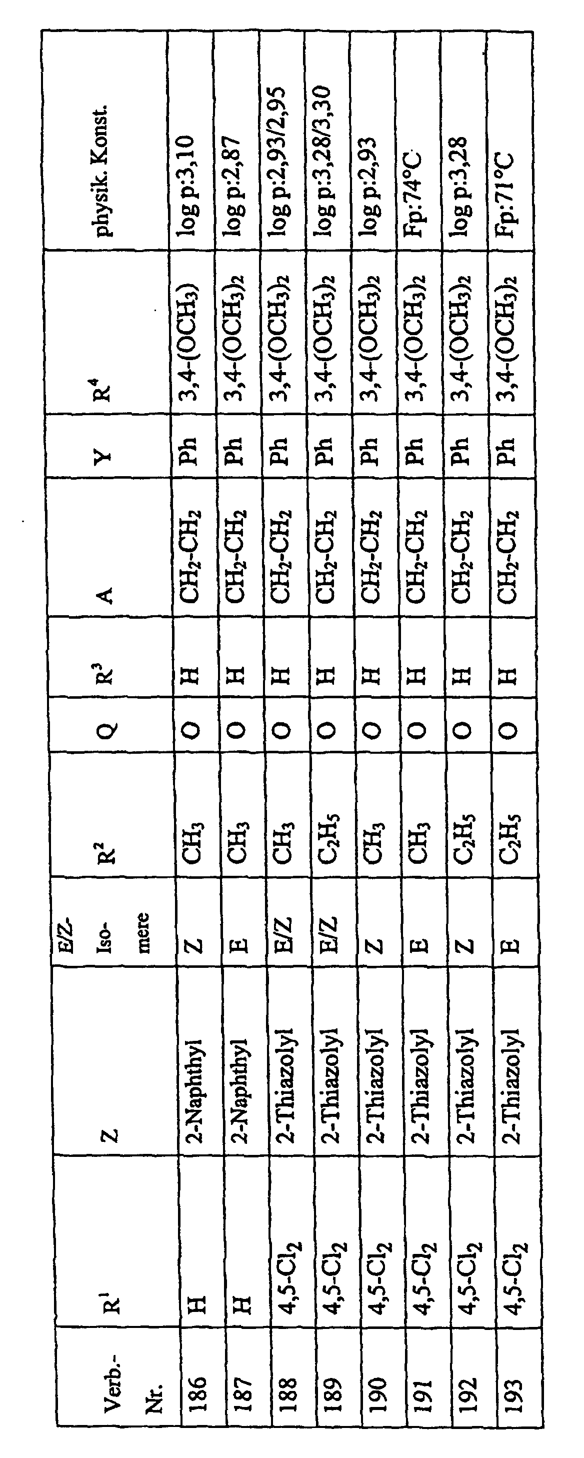

### Beispiel A

### Phytophthora-Test (Tomate) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

In diesem Test zeigen die erfindungsgemäßen Verbindungen 24, 41 und 42 bei einer Wirkstoffkonzentration von 100 ppm in der Spritabrühe einen Wirkungsgrad von mehr als 80 %.

### Beispiel B

### Plasmopara-Test (Rebe) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 21°C und 90 % Luftfeuchtigkeit aufgestellt. Die Pflanzen werden dann angefeuchtet und 1 Tag in eine Feuchtekammer gestellt.

6 Tage nach der Inokulation erfolgt die Auswertung.

In diesem Test zeigen die erfindungsgemäßen Verbindungen 2, 24, 41, 42, 73, 77, 81, 82, 91, 96, 98, 99, 103, 110, 114, 117, 122, 123, 124, 126, 127, 131, 132, 136, 142, 143, 144, 146, 147, 170, 171 und 173 bei einer Wirkstoffkonzentration von 100 ppm in der Spritzbrühe einen Wirkungsgrad von mehr als 80 %.

## Patentansprüche

1. Verbindungen der Formel (I) 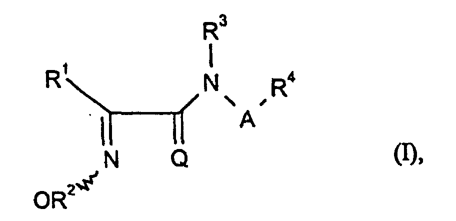 in welcher
A für unsubstituiertes geradkettiges Alkylen steht,
Q fiir Sauerstoff oder Schwefel stehen,
R¹ für jeweils gegebenenfalls substituiertes Cycloalkyl, Cycloalkenyl, Aryl oder Heterocyclyl steht,
R², R³ gleich oder verschieden sind und jeweils für Wasserstoff oder jeweils gegebenenfalls substituiertes Alkyl, Alkenyl, Alkinyl oder Cycloalkyl stehen,
R⁴ für jeweils gegebenenfalls substituiertes Cycloalkyl, Cycloalkenyl, Aryl oder Heterocyclyl steht.

2. Verbindungen der Formel (I) gemäß Anspruch 1, in welcher
A für unsubstituiertes geradkettiges Alkylen mit 1 bis 6 Kohlenstoffatomen steht,
Q fiir Sauerstoff oder Schwefel steht,
R¹ für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedem steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
R² für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) oder gegebenenfalls substituiertes Phenyl, substituiertes Alkyl mit 1 bis 4 Kohlenstoffatomen, Alkenyl, Alkinyl mit jeweils 2 bis 4 Kohlenstoffatomen oder Cycloalkyl mit 3 bis 6 Kohlenstoffatomen steht,
R³ fiir Wasserstoff oder Alkyl mit 1 bis 4 Kohlenstoffatomen steht,
R⁴ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

3. Verbindungen der Formel (I), gemäß Anspruch 1, in welcher
A für Methylen, 1,2-Ethylen, 1,3-Propylen oder 1,4-Butylen steht,
Q für Sauerstoff oder Schwefel steht,
R¹ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylainino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy;
R² fiir Wasserstoff oder für Methyl, Ethyl, n- oder i-Propyl, n-, i-, s- oder t-Butyl oder für Benzyl oder Propargyl oder Allyl steht,
R³ für Wasserstoff oder für Methyl oder Ethyl steht,
R⁴ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlonnethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

4. Verbindungen der Formel (I) gemäß Anspruch 1, in welcher
A fiir Methylen, 1,2-Ethylen, 1,3-Propylen oder 1,4-Butylen steht,
Q für Sauerstoff oder Schwefel steht,
_{R}1 für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
R² für Wasserstoff, Methyl oder Ethyl, n-, i-Propyl, n-, i-, s- oder t-Butyl oder für Benzyl oder Allyl steht,
R³ fiir Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofüranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

5. Verbindungen der Formel (I), in welcher
A für Methylen, 1,2-Ethylen oder 1,3-Propylen steht,
Q fiir Sauerstoff steht,
R¹ für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder fiir jeweils gegebenenfalls durch Fluor substituiertes 3,4-Methylen- und Ethylendioxo, Propan-1,3-diyl und Butan-1,4-diyl substituiertes Phenyl steht oder fiir Naphthyl, Benzofuranyl oder Benzothienyl steht,
R² für Wasserstoff, Methyl oder Ethyl, für n- oder i-Propyl, für n-, i-, s- oder t-Butyl oder für Benzyl oder Allyl steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

6. Schädlingsbekämpfungsmittel, **gekennzeichnet durch** einen Gehalt an mindestens einer Verbindung der Formel (I) nach Anspruch 1.

7. Verfahren zur Bekämpfung von Schädlingen, **dadurch gekennzeichnet, daß** man Verbindungen der Formel (I) nach Anspruch 1 auf Schädlinge und/oder ihren Lebensraum einwirken läßt.

8. Verwendung von Verbindungen der Formel (I) nach den Ansprüchen 1 bis 5 zur Bekämpfung von Schädlingen.

9. Verfahren zur Herstellung von Schädlingsbekämpfungsmitteln, **dadurch gekennzeichnet, daß** man Verbindungen der Formel (I) nach den Ansprüchen 1 bis 5 mit Streckmitteln und/oder oberflächenaktiven Mitteln vermischt.

10. Verfahren zur Herstellung von Verbindungen der Formel 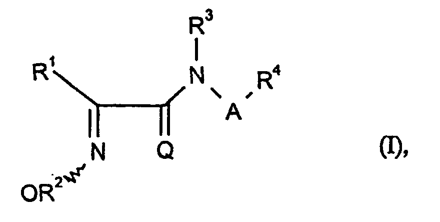 in welcher
A, Q, R¹, R², R³ und R⁴ die in Anspruch 1 angegebenen Bedeutungen haben,
**dadurch gekennzeichnet, daß** man Carbonsäurederivate der allgemeinen Formel (II) 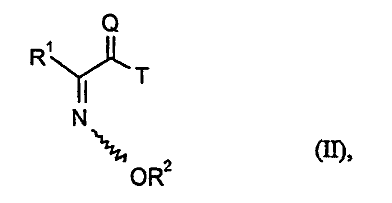 in welcher
R¹, R² und Q die oben angegebene Bedeutung haben und
T für Hydroxy, Halogen oder Alkoxy steht,
mit einem Amin der allgemeinen Formel (III) 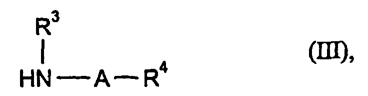 in welcher
R³, R⁴ und A die oben angegebene Bedeutung haben,
- oder mit einem Hydrogenhalogenid hiervon -
gegebenenfalls in Gegenwart eines Säureakzeptors, gegebenenfalls in Gegenwart eines Kondensationsmittels und gegebenenfalls in Gegenwart eines Verdünnungsmittels umsetzt.

11. Verbindungen der allgemeinen Formel (Ia) 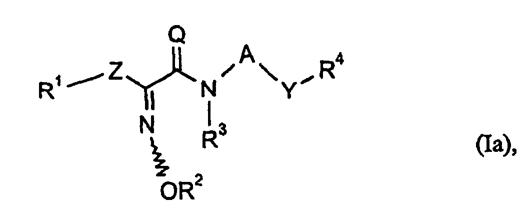 in welcher die Substituenten die in der nachstehenden Tabelle genannten Bedeutungen haben

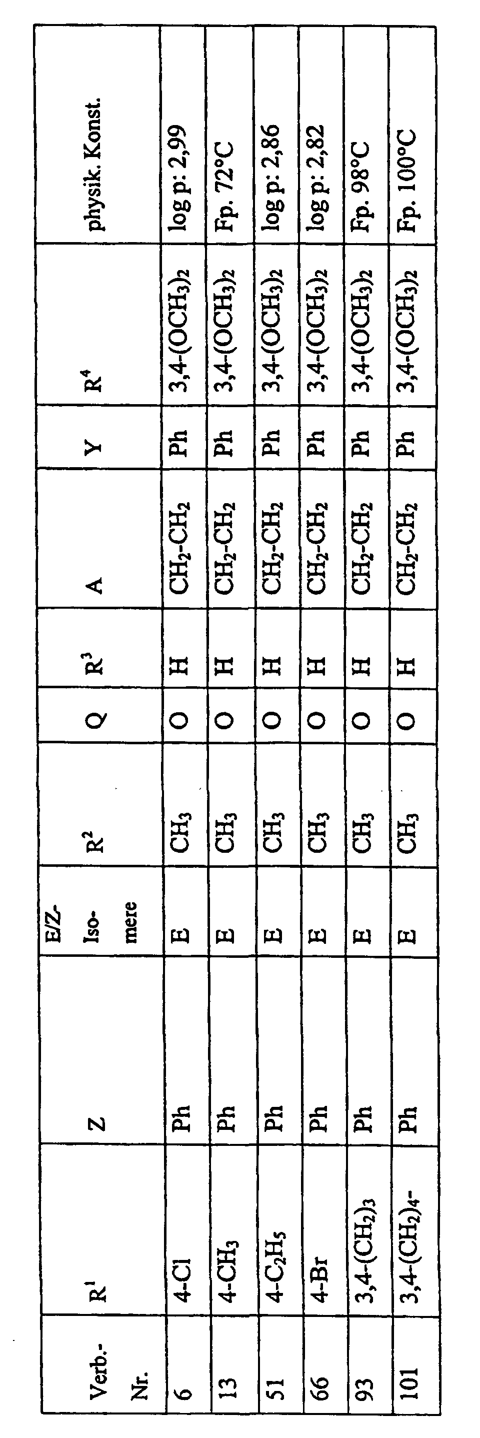

## Claims

1. Compounds of the formula (I) 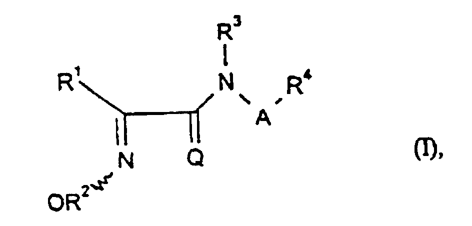 in which
A represents unsubstituted straight-chain alkylene,
Q represents oxygen or sulphur,
R¹ represents in each case optionally substituted cycloalkyl, cycloalkenyl, aryl or heterocyclyl,
R² and R³ are identical or different and in each case represent hydrogen or in each case optionally substituted alkyl, alkenyl, alkinyl or cycloalkyl, and
R⁴ represents in each case optionally substituted cycloalkyl, cycloalkenyl, aryl or heterocyclyl.

2. Compounds of the formula (I) according to Claim 1, in which
A represents unsubstituted straight-chain alkylene having 1 to 6 carbon atoms,
Q represents oxygen or sulphur,
R¹ represents aryl, cycloalkyl or cycloalkenyl having 3 to 7 carbon atoms or heterocyclyl having 3 to 12 ring members, in each case optionally mono- or polysubstituted by identical or different substituents, the possible substituents preferably being chosen from the following list:
halogen, cyano, nitro, amino, hydroxyl, formyl, carboxyl, carbamoyl or thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroxyiminoalkyl or alkoximinoalkyl having in each case 1 to 8 carbon atoms in the individual alkyl parts;
in each case divalent alkylene or dioxyalkylene having in each case 1 to 6 carbon atoms and in each case optionally mono- or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
and substituted aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, in each case optionally mono- or polysubstituted in an identical or different manner by
halogen, cyano and/or straight-chain or branched alkyl having 1 to 4 carbon atoms
and/or straight-chain or branched halogenoalkyl having 1 to 4 carbon atoms and 1 to 9 identical or different halogen atoms
and/or straight-chain or branched alkoxy or alkylthio having 1 to 4 carbon atoms
and/or straight-chain or branched halogenoalkoxy or halogenoalkylthio having 1 to 4 carbon atoms and 1 to 9 identical or different halogen atoms
and/or in each case divalent alkylene or dioxyalkylene having in each case 1 to 6 carbon atoms and optionally mono- or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms,
R² represents hydrogen, or represents alkyl having 1 to 4 carbon atoms, alkenyl or alkinyl having in each case 2 to 4 carbon atoms or cycloalkyl having 3 to 6 carbon atoms, in each case optionally mono- or polysubstituted in an identical or different manner by halogen, cyano, hydroxyl, amino, C₁-C₄-alkoxy, C₁-C₄-alkylthio, C₁-C₄-alkylsulphinyl or C₁-C₄-alkylsulphonyl (which can in each case be optionally substituted by halogen) or by optionally substituted phenyl,
R³ represents hydrogen or alkyl having 1 to 4 carbon atoms,
R⁴ represents aryl, cycloalkyl or cycloalkenyl having 3 to 8 carbon atoms or heterocyclyl having 3 to 12 ring members, in each case optionally mono- or polysubstituted by identical or different substituents, the possible substituents preferably being chosen from the following list:
halogen, cyano, nitro, amino, hydroxyl, formyl, carboxyl, carbamoyl or thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl parts;
in each case divalent alkylene or dioxyalkylene having in each case 1 to 6 carbon atoms and in each case optionally mono- or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
and substituted aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, in each case optionally mono- or polysubstituted in an identical or different manner by
halogen, cyano and/or straight-chain or branched alkyl having 1 to 4 carbon atoms
and/or straight-chain or branched halogenoalkyl having 1 to 4 carbon atoms and 1 to 9 identical or different halogen atoms
and/or straight-chain or branched alkoxy or alkylthio having 1 to 4 carbon atoms
and/or straight-chain or branched halogenoalkoxy or halogenoalkylthio having 1 to 4 carbon atoms and 1 to 9 identical or different halogen atoms
and/or in each case divalent alkylene or dioxyalkylene having in each case 1 to 6 carbon atoms and optionally mono- or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms.

3. Compounds of the formula (I) according to Claim 1, in which
A represents methylene, 1,2-ethylene, 1,3-propylene or 1,4-butylene,
Q represents oxygen or sulphur,
R¹ represents cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, in each case optionally mono- to trisubstituted, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl), methylenedioxy or ethylenedioxy, in each case optionally mono- or polysubstituted in an identical or different manner by fluorine, chlorine, methyl, trifluoromethyl, ethyl or nor i-propyl;
cyclopropyl, cyclobutyl, cyclopentyl or cyclohexyl;
phenyl, benzyl, phenoxy and benzyloxy which are optionally substituted by the abovementioned substituents,
R² represents hydrogen, or represents methyl, ethyl, n- or i-propyl or n-, i-, s- or t-butyl, or represents benzyl or propargyl or allyl,
R³ represents hydrogen, or represents methyl or ethyl,
R⁴ represents cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, in each case optionally mono- to trisubstituted, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl), methylenedioxy or ethylenedioxy, in each case optionally mono- or polysubstituted in an identical or different manner by fluorine, chlorine, methyl, trifluoromethyl, ethyl or nor i-propyl;
cyclopropyl, cyclobutyl, cyclopentyl or cyclohexyl.

4. Compounds of the formula (I) according to Claim 1, in which
A represents methylene, 1,2-ethylene, 1,3-propylene or 1,4-butylene,
Q represents oxygen or sulphur,
R¹ represents cyclobutyl, cyclopentyl or cyclohexyl, in each case optionally mono- to hexasubstituted, preferred substituents being those mentioned below;
or represents phenyl, naphthyl, furyl, benzofuranyl, thienyl, benzothienyl, pyridyl, quinolyl, tetrahydrofuryl or perhydropyranyl, in each case optionally mono- to trisubstituted, the possible substituents preferably being chosen from the following list:
fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl), methylenedioxy or ethylenedioxy, in each case optionally mono- or polysubstituted in an identical or different manner by fluorine, chlorine, methyl, trifluoromethyl, ethyl or nor i-propyl;
cyclopropyl, cyclopentyl or cyclohexyl;
phenyl, phenoxy or benzyloxy optionally substituted by the abovementioned substituents;
R² represents hydrogen, methyl, ethyl, n- or i-propyl or n-, i-, s- or t-butyl, or represents benzyl or allyl,
R³ represents hydrogen or methyl,
R⁴ represents cyclopropyl, cyclobutyl, cyclopentyl or cyclohexyl, in each case optionally mono- to hexasubstituted, preferred substituents being those mentioned below;
or represent phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, in each case optionally mono- to trisubstituted, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl), methylenedioxy or ethylenedioxy, in each case optionally mono- or polysubstituted in an identical or different manner by fluorine, chlorine, methyl, trifluoromethyl, ethyl or nor i-propyl,
cyclopropyl, cyclobutyl, cyclopentyl or cyclohexyl.

5. Compounds of the formula (I) in which
A represents methylene, 1,2-ethylene or 1,3-propylene,
Q represents oxygen,
R¹ represents phenyl, thienyl or furanyl, which are optionally mono- or disubstituted by bromine, chlorine, fluorine, nitro, methylsulphonyl, phenyl, phenyloxy, benzyloxy, cyclopropyl, cyclohexyl, methyl, ethyl, n- or i-propyl, n-, i-, s- or t-butyl, n-pentyl, n-hexyl, n-heptyl, methoxy, ethoxy, trifluoromethyl, trifluoromethoxy and/or methylthio, or represents phenyl which is substituted by in each case optionally fluorine-substituted 3,4-methylene- and ethylenedioxo, propane-1,3-diyl and butane-1,4-diyl, or represents naphthyl, benzofuranyl or benzothienyl,
R² represents hydrogen, methyl or ethyl, or represents n- or i-propyl, or represents n-, i-, s- or t-butyl, or represents benzyl or allyl,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl or quinolyl, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, in each case optionally mono- or polysubstituted in an identical or different manner by fluorine, chlorine, methyl, trifluoromethyl, ethyl or n- or i-propyl.

6. Compositions for combating pests, **characterized by** a content of at least one compound of the formula (I) according to Claim 1.

7. Method of combating pests, **characterized in that** compounds of the formula (I) according to Claim 1 are allowed to act on pests and/or their environment.

8. Use of compounds of the formula (I) according to Claims 1 to 5 for combating pests.

9. Process for the preparation of compositions for combating pests, **characterized in that** compounds of the formula (I) according to Claims 1 to 5 are mixed with extenders and/or surface-active agents.

10. Process for the preparation of compounds of the formula 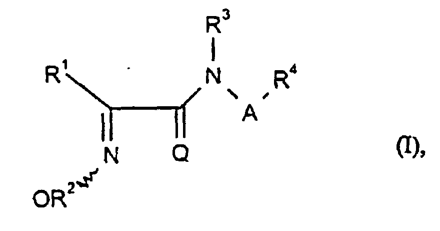 in which
A, Q, R¹, R², R³ and R⁴ have the meanings given in Claim 1,
**characterized in that** carboxylic acid derivatives of the general formula (II) 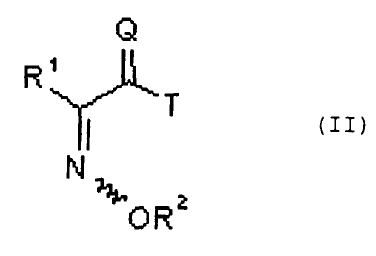 in which
R¹, R² and Q have the abovementioned meaning and
T represents hydroxyl, halogen or alkoxy,
are reacted with an amine of the general formula (III) 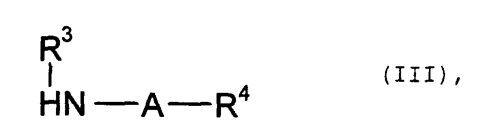 in which
R³, R⁴ and A have the abovementioned meaning
- or with a hydrogen halide thereof -
if appropriate in the presence of an acid acceptor, if appropriate in the presence of a condensing agent and if appropriate in the presence of a diluent.

11. Compounds of the general formula (Ia) 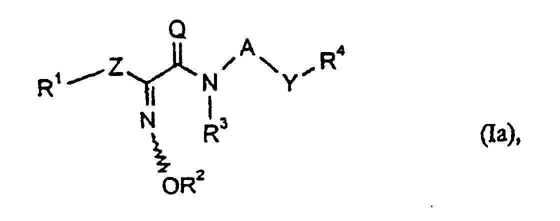 in which the substituents have the meanings mentioned in the following table

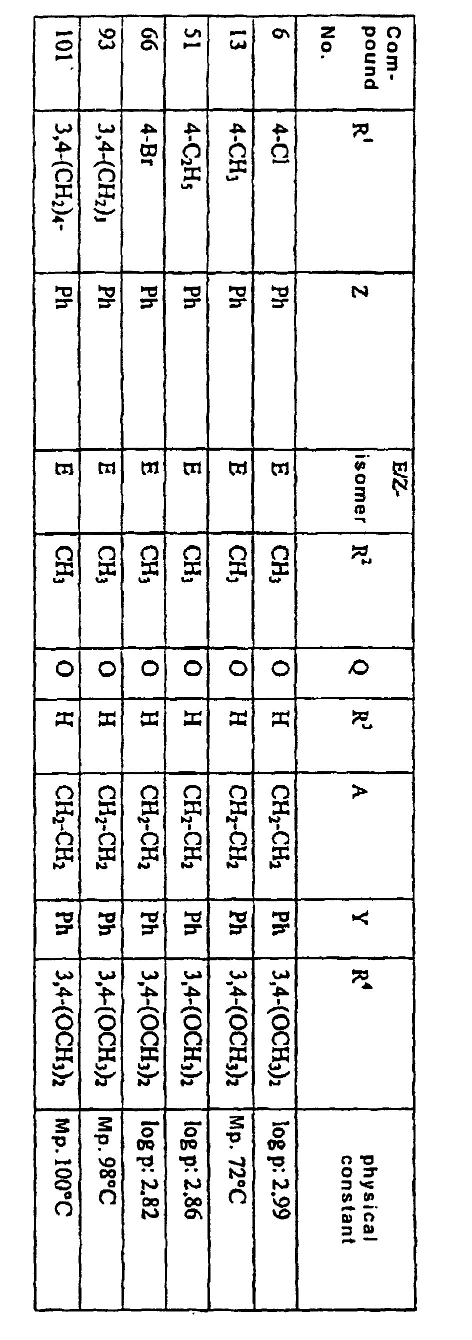

## Revendications

1. Composés de formule (I) 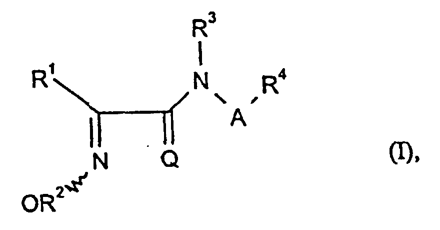 dans laquelle
A est un groupe alkylène linéaire non substitué,
Q représente l'oxygène ou le soufre,
R¹ est un groupe cycloalkyle, cycloalcényle, aryle ou hétérocyclyle, éventuellement substitué dans chaque cas,
R², R³ sont identiques ou différents et chacun d'eux représente l'hydrogène ou un groupe alkyle, alcényle, alcynyle ou cycloalkyle dont chacun est éventuellement substitué,
R⁴ représente un groupe cycloalkyle, cycloalcényle, aryle ou hétérocyclyle dont chacun est éventuellement substitué.

2. Composés de formule (I) suivant la revendication 1, dans laquelle
A est un groupe alkylène linéaire non substitué ayant 1 à 6 atomes de carbone,
Q représente l'oxygène ou le soufre,
R¹ est un groupe aryle, cycloalkyle ou cycloalcényle ayant 3 à 7 atomes de carbone, chacun portant éventuellement un ou plusieurs substituants identiques ou différents, ou un groupe hétérocyclyle à noyau de 3 à 12 atomes, les substituants possibles étant choisis de préférence parmi ceux qui sont énumérés ci-après :
halogéno, cyano, nitro, amino, hydroxy, formyle, carboxy, carbamoyle, thiocarbamoyle ;
alkyle, alkoxy, alkylthio, alkylsulfinyle ou alkylsulfonyle étant chacun linéaire ou ramifié et ayant 1 à 6 atomes de carbone ;
alcényle ou alcényloxy étant chacun linéaire ou ramifié et ayant chacun 2 à 6 atomes de carbone ; halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ;
halogénalcényle ou halogénalcényloxy étant chacun linéaire ou ramifié et ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle étant chacun linéaire ou ramifié et ayant chacun 1 à 8 atomes de carbone dans les parties alkyle individuelles ;
alkylène ou dioxyalkylène ayant chacun deux liaisons et 1 à 6 atomes de carbone, chacun portant éventuellement un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, portant éventuellement un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
R² représente l'hydrogène ou un groupe alkyle ayant 1 à 4 atomes de carbone, alcényle, alcynyle ayant chacun 2 à 4 atomes de carbone ou cycloalkyle ayant 3 à 6 atomes de carbone, portant chacun le cas échéant un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun d'eux pouvant être éventuellement substitué par un halogène) ou phényle éventuellement substitué,
R³ représente l'hydrogène ou un groupe alkyle ayant 1 à 4 atomes de carbone,
R⁴ représente un groupe, portant éventuellement un ou plusieurs substituants, identiques ou différents, aryle, cycloalkyle ou cycloalcényle ayant 3 à 8 atomes de carbone ou hétérocyclyle à noyau de 3 à 12 atomes, les substituants possibles étant choisis de préférence parmi les substituants énumérés ci-après :
halogéno, cyano, nitro, amino, hydroxy, formyle, carboxy, carbamoyle, thiocarbamoyle ;
alkyle, alkoxy, alkylthio, alkylsulfinyle ou alkylsulfonyle, étant chacun linéaire ou ramifié et ayant chacun 1 à 6 atomes de carbone ;
alcényle ou alcényloxy étant chacun linéaire ou ramifié et ayant chacun 2 à 6 atomes de carbone ; halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ;
halogénalcényle ou halogénalcényloxy étant chacun linéaire ou ramifié et ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle étant chacun linéaire ou ramifié et ayant chacun 1 à 6 atomes de carbone dans les parties alkyle individuelles ;
alkylène ou dioxyalkylène ayant chacun deux liaisons et chacun 1 à 6 atomes de carbone, portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents.

3. Composés de formule (I) suivant la revendication 1, dans laquelle
A est un groupe méthylène, 1,2-éthylène, 1,3-propylène ou 1,4-butylène,
Q représente l'oxygène ou le soufre,
R¹ est un groupe cyclopropyle, cyclobutyle, cyclopentyle ou cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis de préférence dans l'énumération suivante :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthyl-sulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxy-carbonyle, éthoxy-carbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
triméthylène(propane-1,3-diyle), tétraméthylène(butane-1,4-diyle), méthylène-dioxy ou éthylène-dioxy ayant chacun deux liaisons, portant chacun le cas échéant un ou plusieurs substituants, identiques ou différents, fluoro, chloro, méthyle, trifluorométhyle, éthyle, n-propyle ou isopropyle ;
cyclopropyle, cyclobutyle, cyclopentyle ou cyclohexyle ;
phényle, benzyle, phénoxy, benzyloxy portant éventuellement les substituants mentionnés ci-dessus ;
R² représente l'hydrogène ou un groupe méthyle, éthyle, n-propyle, isopropyle, n-butyle, isobutyle, sec.-butyle, tertiobutyle, benzyle, propargyle ou allyle,
R³ est l'hydrogène ou le groupe méthyle ou éthyle,
R⁴ représente un groupe cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis de préférence dans l'énumération suivante :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxy-carbonyle, éthoxy-carbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
triméthylène(propane-1,3-diyle), tétraméthylène(butane-1,4-diyle), méthylène-dioxy, éthylène-dioxy, cyclopropyle, cyclobutyle, cyclopentyle ou cyclohexyle ayant chacun deux liaisons et portant chacun le cas échéant un ou plusieurs substituants, identiques ou différents, fluoro,
chloro, méthyle, trifluorométhyle, éthyle, n-propyle ou isopropyle.

4. Composés de formule (I) suivant la revendication 1, dans laquelle
A est un groupe méthylène, 1,2-éthylène, 1,3-propylène ou 1,4-butylène,
Q représente l'oxygène ou le soufre,
R¹ est un groupe cyclobutyle, cyclopentyle ou cyclohexyle portant chacun le cas échéant 1 à 6 substituants, avec comme substituants appréciés ceux qui sont mentionnés ci-dessous ; un groupe phényle, naphtyle, furyle, benzofurannyle, thiényle, benzothiényle, pyridyle, quinolyle, tétrahydrofuryle ou perhydropyrannyle portant chacun le cas échéant 1 à 3 substituants, les substituants possibles étant choisis de préférence dans l'énumération suivante :
fluor, chlore, brome, cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxy-carbonyle, éthoxycarbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle ;
triméthylène(propane-1,3-diyle), tétraméthylène(butane-1,4-diyle), méthylène-dioxy ou éthylène-dioxy ayant chacun deux liaisons et portant chacun le cas échéant un ou plusieurs substituants, identiques ou différents, fluor,
chlore, méthyle, trifluorométhyle, éthyle, n-propyle ou isopropyle ;
cyclopropyle, cyclopentyle, cyclohexyle ;
phényle, phénoxy, benzyloxy portant éventuellement les substituants mentionnés ci-dessus ;
R² représente l'hydrogène, un groupe méthyle, éthyle, n-propyle, isopropyle, n-butyle, isobutyle, sec.-butyle, tertiobutyle, benzyle ou allyle,
R³ est l'hydrogène ou le groupe méthyle,
R⁴ représente un groupe cyclopropyle, cyclobutyle, cyclopentyle ou cyclohexyle portant chacun le cas échéant 1 à 6 substituants, avec comme substituants appréciés les substituants mentionnés ci-dessous ; phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis de préférence dans l'énumération ci-dessous :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxy-carbonyle, éthoxy-carbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
triméthylène(propane-1,3-diyle), tétraméthylène(butane-1,4-diyle), méthylène-dioxy ou éthylène-dioxy ayant chacun deux liaisons et portant chacun le cas échéant un ou plusieurs substituants, identiques ou différents, fluoro,
chloro, méthyle, trifluorométhyle, éthyle, n-propyle ou isopropyle,
cyclopropyle, cyclobutyle, cyclopentyle ou cyclohexyle.

5. Composés de formule (I), dans laquelle
A est un groupe méthylène, 1,2-éthylène ou 1,3-propylène,
Q est l'oxygène,
R¹ représente un groupe phényle, thiényle ou furannyle portant éventuellement 1 ou 2 substituants bromo, chloro, fluoro, nitro, méthylsulfonyle, phényle, phényloxy, benzyloxy, cyclopropyle, cyclohexyle, méthyle éthyle, n-propyle, isopropyle, n-butyle, isobutyle, sec.-butyle, tertiobutyle, n-pentyle, n-hexyle, n-heptyle, méthoxy, éthoxy, trifluorométhyle, trifluorométhoxy et/ou méthylthio ou un groupe phényle portant un substituant 3,4-méthylènedioxo, 3,4-éthylène-dioxo, propane-1,3-diyle et butane-1,4-diyle éventuellement substitué dans chaque cas par du fluor, ou un groupe naphtyle, benzofurannyle ou benzothiényle,
R² représente l'hydrogène, un groupe méthyle, éthyle, n-propyle, isopropyle, n-butyle, isobutyle, sec.-butyle, tertiobutyle ou un groupe benzyle ou allyle,
R³ représente l'hydrogène ou le groupe méthyle,
R⁴ est un groupe cyclohexyle ou un groupe phényle, thiényle, furyle, benzofuryle, benzothiényle, pyridyle, pyrimidinyle, naphtyle, quinolyle portant éventuellement 1 à 3 substituants, les substituants possibles étant de préférence choisis dans l'énumération ci-dessous :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxy-carbonyle, éthoxy-carbonyle, méthylsulfonyloxy, éthylsulfonyloxy, hydroximinométhyle, hydroximino-éthyle, méthoximinométhyle, éthoximinométhyle, méthoximino-éthyle ou éthoximino-éthyle,
un groupe triméthylène(propane-1,3-diyle), tétraméthylène(butane-1,4-diyle), méthylène-dioxy ou éthylène-dioxy ayant chacun deux liaisons et portant chacun le cas échéant un ou plusieurs substituants, identiques ou différents, fluoro, chloro, méthyle, trifluorométhyle, éthyle, n-propyle ou isopropyle.

6. Composition pesticide, **caractérisée par** une teneur en au moins un composé de formule (I) suivant la revendication 1.

7. Procédé pour combattre des parasites, **caractérisé en ce qu'**on fait agir des composés de formule (I) suivant la revendication 1 sur les parasites et/ou sur leur milieu.

8. Utilisation de composés de formule (I) suivant les revendications 1 à 5 pour combattre des parasites.

9. Procédé de préparation de compositions pesticides, **caractérisé en ce qu'**on mélange des composés de formule (I) suivant les revendications 1 à 5 avec des diluants et/ou des agents tensioactifs.

10. Procédé de production de composés de formule 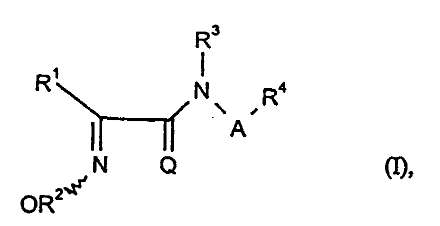 dans laquelle
A, Q, R¹, R², R³ et R⁴ ont les définitions indiquées dans la revendication 1,
**caractérisé en ce qu'**on fait réagir des dérivés d'acides carboxyliques de formule générale (II) 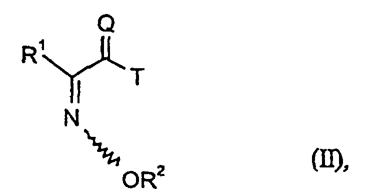 dans laquelle
R¹, R² et Q ont la définition indiquée ci-dessus et
T est un groupe hydroxy, halogène ou alkoxy,
avec une amine de formule générale (III) 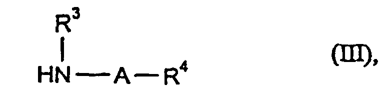 dans laquelle
R³, R⁴ et A ont la définition indiquée ci-dessus,
- ou avec un halogénhydrate de cette amine -
le cas échéant en présence d'un accepteur d'acide, en présence éventuelle d'un agent de condensation et le cas échéant en présence d'un diluant.

11. Composés de formule générale (Ia) 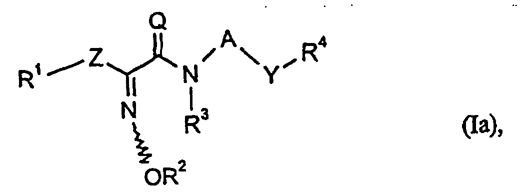 dans laquelle les substituants ont les définitions mentionnées dans le tableau ci-après
